# FASCICULE DE BREVET EUROPEEN

(11) **EP 4 452 950 B1**
(45) Date de publication et mention de la délivrance du brevet: **11.02.2026**
(21) Numéro de dépôt: 22840089.1
(22) Date de dépôt: 19.12.2022
(51) Int. Cl.: C07D 233/61

(54) **PROCEDE DE SYNTHESE D'UNE OXIME COMPRENANT UN GROUPEMENT IMIDAZOLE ET PROCEDE DE SYNTHESE D'UN OXYDE DE NITRILE A PARTIR DE LADITE OXIME**
VERFAHREN ZUR SYNTHESE EINES OXIMS MIT EINER IMIDAZOLGRUPPE UND VERFAHREN ZUR SYNTHESE EINES NITRILOXIDS AUS DIESEM OXIM
METHOD FOR SYNTHESIZING AN OXIME COMPRISING AN IMIDAZOLE GROUP AND METHOD FOR SYNTHESIZING A NITRILE OXIDE FROM SAID OXIME

(30) Priorité: 21.12.2021 FR 2114104
(43) Date de publication de la demande: 30.10.2024
(73) Titulaire: COMPAGNIE GENERALE DES ETABLISSEMENTS MICHELIN, 63000 Clermont-Ferrand (FR)
(72) Inventeur: JEAN-BAPTISTE-DIT-DOMINIQUE, François, 63040 Clermont-Ferrand CEDEX 09 (FR); IVANOV, Sergey, 63040 Clermont-Ferrand CEDEX 09 (FR); UGOLNIKOV, Oleg, 63040 Clermont-Ferrand CEDEX 09 (FR); LE ROUX, Stephane, 61150 Écouché les Vallées (FR); JOSEPH, Jean Christophe, 78520 Limay (FR); ROOL, Patrice, 63200 Riom (FR); MICHAUX, Julien, 63200 Riom (FR)
(74) Mandataire: M.F.P. Michelin
(86) Numéro de dépôt international: PCT/EP2022/086547
(87) Numéro de publication internationale: WO 2023/117838

(56) Documents cités:
- WO-A1-2016/135195
- US-A1- 2010 324 273

## Description

La présente invention concerne un procédé de synthèse d'une oxime portant un groupe imidazole (ci-après appelée composé de formule (1)) ainsi qu'un procédé de synthèse d'un oxyde de nitrile portant un groupe imidazole (ci-après appelé composé de formule (X)) via la synthèse du composé de formule (I) selon le procédé de synthèse de l'invention.

Les oxydes de nitrile comportant un groupement imidazole sont utilisés en tant qu'agent de modification d'élastomères diéniques. Ils permettent, une fois greffés sur les doubles liaisons carbone-carbone de ces élastomères diéniques, de modifier leur structure et in fine d'améliorer les propriétés mécaniques des compositions élastomériques comprenant de tels élastomères diéniques greffés et au moins une charge renforçante. Ces oxydes de nitrile sont décrits dans le document WO2015059269.

Actuellement, lors de la synthèse de ces oxydes de nitriles, il est nécessaire de réaliser de longues et nombreuses étapes de séparation, de purification, d'isolement et de séchage de chacun des intermédiaires de synthèse afin d'éviter la présence d'impuretés qui entraînent des réactions secondaires ayant un impact négatif sur la sélectivité de la réaction et sur son rendement.

En outre, le procédé de synthèse de l'art antérieur de ces oxydes de nitrile met en œuvre des matières premières et/ou des solvants qui sont très couteux à leurs achats. Par exemple, la deuxième étape du procédé de l'art antérieur décrit dans des exemples de WO2015059269 met en œuvre une étape de formylation en présence de tétrachlorure de titane et d'éther de dichlorométhyle et méthyle ; la troisième étape s'effectue en présence de N,N-diméthylformamide. Ce dernier composé est utilisé en tant que solvant et en grande quantité. Par ailleurs, son utilisation est de moins en moins recommandable par les réglementations nationales. L'utilisation de ces solvants impose des contraintes supplémentaires en termes de sécurité, de manipulations et de traitement des rejets pour l'environnement rendant ce procédé de synthèse complexe et couteux, notamment pour une synthèse à une échelle industrielle.

Par conséquent, il existe un besoin de fournir un procédé de synthèse d'un composé oxyde de nitrile comportant un groupement imidazole qui soit amélioré. Plus particulièrement, il existe un besoin pour un procédé de synthèse d'un composé oxyde de nitrile comportant un groupement imidazole qui soit applicable à l'échelle industrielle et qui permet de réduire le nombre d'étapes d'isolement des intermédiaires de synthèse, de réduire les temps de cycle, de réduire la quantité des solvants utilisés, de réduire le nombre de solvants différents utilisés, d'augmenter les rendements en limitant la formation de sous-produits et in fine de réduire le coût de production de ces oxydes de nitrile.

La présente invention a pour but de répondre à ce besoin et de fournir un procédé de synthèse amélioré par rapport à celui de l'art antérieur.

Poursuivant ses recherches, la demanderesse a mis au point un nouveau procédé de synthèse d'une oxime portant un groupe imidazole ainsi qu'un procédé de synthèse d'un oxyde de nitrile à partir de ladite oxime synthétisée selon le procédé de l'invention ; ce procédé présentant un rendement de synthèse significativement amélioré par rapport au procédé de l'art antérieur et permettant également de réduire le nombre de solvants et leur quantité.

Ainsi, la présente invention concerne un procédé de synthèse d'un composé de formule (I) 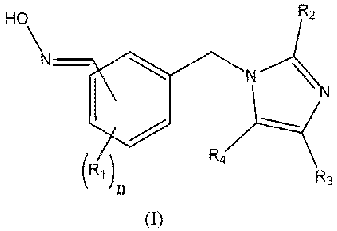 dans laquelle :
- R₁ représente, indépendamment les uns des autres, un alkyle en C1-C6, de préférence un alkyle en C1-C3 ;
- R₂ représente un atome d'hydrogène, un alkyle en C1-C12, de préférence un alkyle en C1-C6, plus préférentiellement un alkyle en C1-C3 ;
- R₃ et R₄, indépendamment l'un de l'autre, représente un atome d'hydrogène, un alkyle en C1-C12, (de préférence un alkyle en C1-C6, plus préférentiellement un alkyle en C1-C3) ou forment ensemble avec les atomes de carbone auxquels ils se rattachent un cycle , notamment un cycle aromatique, de préférence le phényle; et
- n un nombre entier égal à 0, 1, 2, 3 ou 4 ;
ledit procédé de synthèse comprenant les étapes successives A1, A2, A3 suivantes :
- une étape d'obtention A1 d'un composé intermédiaire de formule (V) par une réaction de halogénométhylation d'un aldéhyde aromatique de formule (VI) en présence d'un agent de halogénométhylation selon le schéma réactionnel suivant : 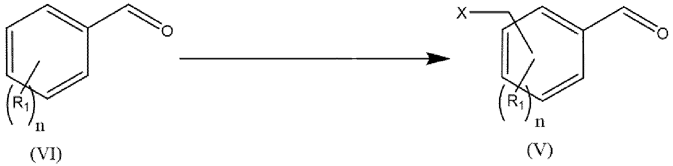 avec R₁ et n tels que définis ci-dessus ; et X étant un atome d'halogène, de préférence un atome de brome ou de chlore, plus préférentiellement un atome de chlore ;
- une étape d'obtention A2 d'un composé intermédiaire de formule (III) par substitution nucléophile du composé intermédiaire de formule (V) précédent avec un composé imidazole de formule (IV) selon le schéma réactionnel suivant : 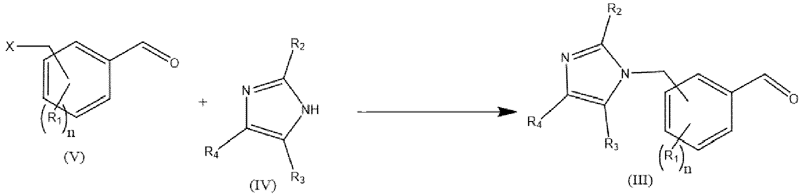 avec n, X, R₁, R₂, R₃ et R₄ tels que définis ci-dessus ;
- une étape d'obtention A3 du composé de formule (I) par une réaction de condensation du composé intermédiaire de formule (III) précédent avec l'hydroxylamine (II) selon le schéma réactionnel suivant :] 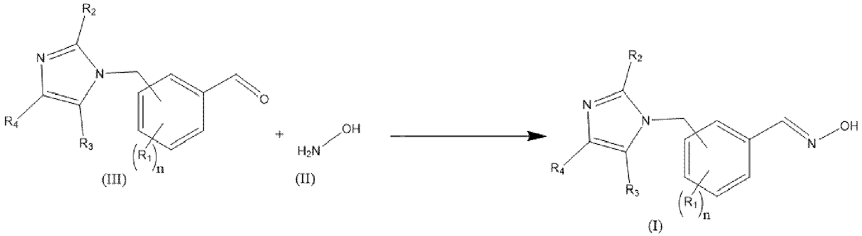
   avec R₁, R₂, R₃, R₄ et n tels que définis ci-dessus ; et
   les étapes A1, A2, A3 étant effectuées sans isolement d'au moins un composé intermédiaire choisi dans le groupe formé par le composé intermédiaire de formule (III) et le composé intermédiaire de formule (V).

Préférentiellement, l'agent d'halogénométhylation est préchauffé à une température T1 comprise dans un domaine allant de 23°C à 50°C, plus préférentiellement dans un domaine allant de 30°C à 45°C, avant sa mise en contact avec le composé de formule (VI).

Préférentiellement, le composé de formule (VI) est ajouté en une seule fois après le préchauffage de l'agent d'halogénométhylation.

Préférentiellement, l'agent d'halogénométhylation est un mélange d'un donneur de formol et d'acide chlorhydrique.

Préférentiellement, la quantité de donneur de formol est comprise dans un domaine allant de 1 équivalent molaire à 7 équivalents molaires, de préférence de 1,2 équivalents molaires à 6 équivalents molaires, plus préférentiellement encore de 2 équivalents molaires à 6 équivalents molaires par rapport à la quantité de composé de formule (VI).

Préférentiellement, l'étape A1 s'effectue à une température T2 comprise dans un domaine allant de 60°C à 100°C, plus préférentiellement dans un domaine allant de 70°C à 90°C.

Préférentiellement, on ajoute un solvant organique S1 à la fin de l'étape A1 et on récupère la phase organique, le solvant organique S1 étant choisi parmi les solvants halogénés.

Préférentiellement, l'étape A2 s'effectue en présence d'un solvant organique S2 choisi dans le groupe constitué par les solvants halogénés, le diméthylsulfoxyde, l'acétone, les alcools tels que l'isopropanol, les éthers tels que le tétrahydrofurane, l'acétate d'éthyle ; préférentiellement le solvant organique S2 est un solvant organique halogéné, plus préférentiellement un solvant organique chloré.

Préférentiellement, le solvant organique S2 est identique au solvant organique S1.

Préférentiellement, la quantité de composé de formule (IV) est comprise dans un domaine allant de 1,1 équivalents molaires à 6 équivalents molaires, plus préférentiellement allant de 2 équivalents molaires à 4 équivalents molaires par rapport au composé de formule (V).

Préférentiellement, l'étape A2 s'effectue à une température qui est inférieure ou égale à la température de reflux du solvant organique S2.

Préférentiellement, la quantité de solvant organique S2 est d'au moins 0,5 volume par rapport à 1 volume de solvant organique S1, plus préférentiellement est comprise dans un domaine allant de 1 volume à 10 volumes par rapport à un volume de solvant organique S1.

Préférentiellement, l'étape A3 s'effectue dans un solvant organique S3 choisi dans le groupe constitué par les solvants halogénés, l'acétone, les alcools tels que l'isopropanol, les éthers tels que le tétrahydrofurane, l'acétate d'éthyle préférentiellement le solvant organique S3 un solvant organique halogéné, de préférence est un solvant organique chloré.

Préférentiellement, le solvant organique S3 est identique au solvant organique S2.

Les composés de formule (I) obtenus selon le procédé de synthèse de l'invention peuvent être transformés en oxydes de nitrile correspondant tout en conservant un bon rendement de production et en particulier un rendement supérieur au rendement des procédés de synthèse des oxydes de nitrile de l'art antérieur.

L'invention concerne également un procédé de synthèse d'un composé de formule (X), ledit procédé comprenant les étapes suivantes :
- une étape (i) de synthèse du composé de formule (I) selon le procédé décrit ci-dessus,
- une étape (ii) de transformation du composé de formule (I) en composé de formule (X) en présence d'au moins un solvant organique S4 et d'au moins un oxydant selon le schéma réactionnel suivant : 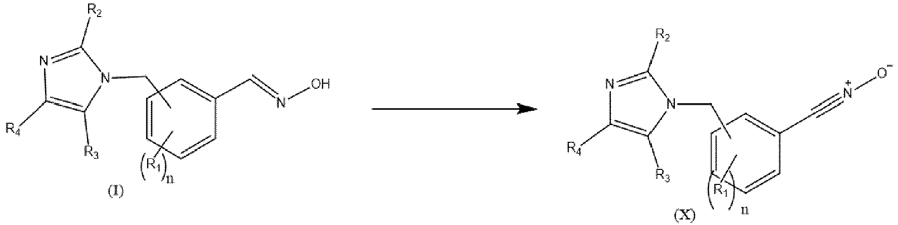 avec :
   - R₁ représente, indépendamment les uns des autres, un alkyle en C1-C6, de préférence un alkyle en C1-C3 ;
   - R₂ représente un atome d'hydrogène, un alkyle en C1-C12, de préférence un alkyle en C1-C6, plus préférentiellement un alkyle en C1-C3 ;
   - R₃ et R₄, indépendamment l'un de l'autre, représente un atome d'hydrogène, un alkyle en C1-C12, (de préférence un alkyle en C1-C6, plus préférentiellement un alkyle en C1-C3) ou forment ensemble avec les atomes de carbone auxquels ils se rattachent un cycle, de préférence un cycle aromatique, de préférence le phényle; et
   - n un nombre entier égal à 0, 1, 2, 3 ou 4 ; et
- une étape (iii) de récupération du composé de formule (X).

Le procédé de synthèse selon l'invention permet ainsi de réduire le nombre d'étapes chimiques par rapport au procédé de synthèse de l'art antérieur, tout en en utilisant des solvants plus acceptables, avec des rendements de synthèse optimisés. Le recours notamment à un aldéhyde aromatique comme matière première de départ et notamment à des solvants appropriés permet d'enchaîner les étapes A1, A2 et A3 sans nécessité d'isoler au moins un intermédiaire de synthèse obtenu lors des étapes A1 et A2. Ainsi, l'halogénométhylation du composé de formule (VI) est réalisée, puis suivie directement par une substitution nucléophile avec un composé de formule (IV), elle-même suivie directement par une réaction de condensation avec l'hydroxylamine aboutissant ainsi à l'oxime de formule (I) avec un niveau de pureté et de rendement satisfaisant et ce sans isolement des composés intermédiaire de formule (V) et/ou de formule (III), de préférence sans isolement des composés intermédiaires de formule (V) et de formule (III). Le procédé de synthèse selon l'invention permet avantageusement d'éviter les étapes de séchage et de limiter les étapes de séparation et de purification de ces composés intermédiaires de synthèse. Finalement, le temps de cycle global est amélioré puisqu'aucun séchage des intermédiaires de synthèse n'est réalisé. Le procédé de synthèse selon l'invention est donc plus économique, plus rapide et plus respectueux de l'environnement.

Le procédé selon l'invention est applicable à l'échelle industrielle et permet notamment d'obtenir des rendements cumulés d'au moins 60% pour l'enchainement sans isolement des intermédiaires des étapes A1 et A2.

En outre, le procédé de synthèse selon l'invention présente également comme avantage, de réduire le nombre et la quantité de solvants et également d'utiliser des solvants communs tels que des solvants alcooliques qui sont plus respectueux de l'environnement et des opérateurs que par exemple le N,N-diméthylformamide.

Dans la présente demande, sauf indication expresse différente, tous les pourcentages (%) indiqués sont des pourcentages (%) en masse.

D'autre part, tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de a jusqu'à b (c'est-à-dire incluant les bornes strictes a et b).

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Evidemment, les composés mentionnés peuvent également provenir du recyclage de matériaux déjà utilisés, c'est-à-dire qu'ils peuvent être, partiellement ou totalement, issus d'un procédé de recyclage, ou encore obtenus à partir de matières premières elles-mêmes issues d'un procédé de recyclage. Sont concernés notamment les polymères, les plastifiants, les charges, etc.

Par « alkyle en C1-Cx », on entend, au sens de la présente invention, une chaîne hydrocarbonée saturée, linéaire ou ramifiée, comportant 1 à x atomes de carbone. A titre d'exemple, on peut citer les groupes méthyle, éthyle, propyle, isopropyle, butyle, isobutyle, sec-butyle, tert-butyle, pentyle ou encore hexyle.

Par « cycle aromatique », on entend, au sens de la présente invention, un groupe hydrocarboné aromatique comportant de 6 à 20 atomes de carbone, et comprenant un ou plusieurs cycles accolés, comme un groupement phényle ou naphtyle. Avantageusement, il s'agit du phényle (aryle en C6).

Par « température ambiante », on entend, au sens de la présente invention, une température comprise dans un domaine allant de 20°C à 25°C, plus préférentiellement encore une température de 23°C.

Les expressions « composé intermédiaire », « intermédiaire de synthèse », « composé intermédiaire de synthèse » sont interchangeables et désignent un composé chimique destiné à être transformé chimiquement pour créer un autre composé. En ce sens, l'intermédiaire ne doit plus être présent dans le produit final, ou du moins seulement à l'état d'impureté résiduelle.

Par « atome d'halogène » on entend au sens de la présente invention, un atome choisi dans le groupe formé par le chlore, le brome, l'iode et le fluor. Plus préférentiellement, il s'agit d'un atome de brome ou de chlore, plus préférentiellement encore un atome de chlore.

Un premier objet de la présente invention concerne un procédé de synthèse d'un composé de formule (I) 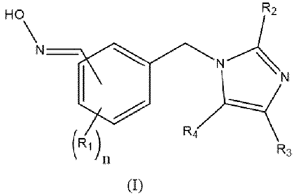 dans laquelle :
- R₁ représente, indépendamment les uns des autres, un alkyle en C1-C6, de préférence un alkyle en C1-C3 ;
- R₂ représente un atome d'hydrogène, un alkyle en C1-C12, de préférence un alkyle en C1-C6, plus préférentiellement un alkyle en C1-C3 ;
- R₃ et R₄, indépendamment l'un de l'autre, représente un atome d'hydrogène, un alkyle en C1-C12, (de préférence un alkyle en C1-C6, plus préférentiellement un alkyle en C1-C3) ou forment ensemble avec les atomes de carbone auxquels ils se rattachent un cycle, notamment un cycle aromatique, de préférence le phényle; et
- n un nombre entier égal à 0, 1, 2, 3 ou 4 ; et
ledit procédé de synthèse comprenant les étapes successives A1, A2, A3 suivantes :
- une étape d'obtention A1 d'un composé intermédiaire de formule (V) par une réaction de halogénométhylation d'un aldéhyde aromatique de formule (VI) en présence d'un agent de halogénométhylation selon le schéma réactionnel suivant : 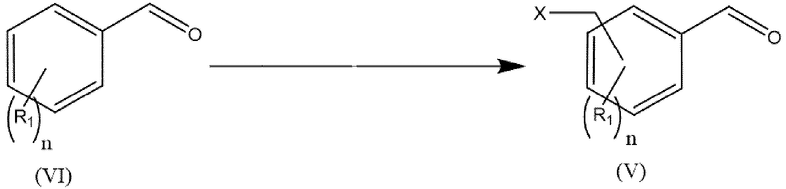 avec R₁ et n tels que définis ci-dessus ; et X étant un atome d'halogène, de préférence un atome de brome ou de chlore, plus préférentiellement un atome de chlore ;
- une étape d'obtention A2 d'un composé intermédiaire de formule (III) par substitution nucléophile du composé intermédiaire de formule (V) précédent avec un composé imidazole de formule (IV) selon le schéma réactionnel suivant : 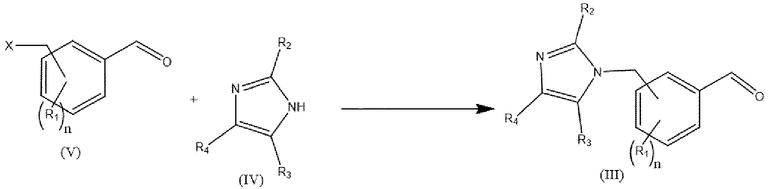 avec n, X, R₁, R₂, R₃ et R₄ tels que définis ci-dessus ;
- une étape d'obtention A3 du composé de formule (I) par une réaction de condensation du composé intermédiaire de formule (III) précédent avec l'hydroxylamine (II) selon le schéma réactionnel suivant : 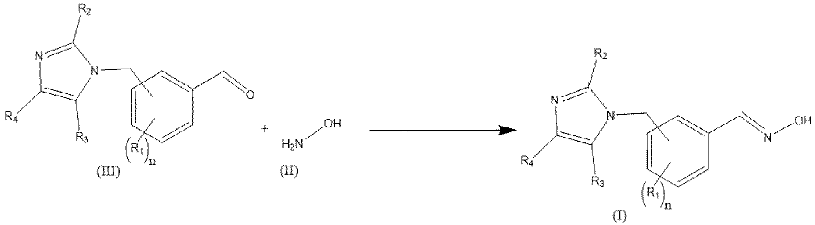
   avec R₁, R₂, R₃, R₄ et n tels que définis ci-dessus ; et
   les étapes A1, A2, A3 étant effectuées sans isolement d'au moins un composé intermédiaire choisi dans le groupe formé par le composé intermédiaire de formule (III) et le composé intermédiaire de formule (V).

Préférentiellement, le procédé de l'invention comprend un enchaînement des réactions A1, A2 et A3, dans lequel le composé intermédiaire de synthèse (V) issu de la réaction d'halogénométhylation (étape A1) n'est ni isolé, ni séché avant de procéder à la réaction de substitution nucléophile avec un composé imidazole de formule (IV) (étape A2). Préférentiellement, le composé intermédiaire de synthèse de formule (III) issu de la réaction de substitution nucléophile avec un composé imidazole (étape A2) n'est ni isolé, ni séché avant de procéder à la réaction de condensation avec l'hydroxylamine (étape A3). Plus préférentiellement encore, ni le composé intermédiaire de synthèse de formule (V) ni le composé intermédiaire de synthèse de formule (III) ne sont isolés ou séchés lors de l'enchaînement des étapes A1 vers A2 et A2 vers A3.

L'expression « sans isolement » signifie au sens de la présente invention, que dans un procédé de synthèse ou un enchainement de réaction, un intermédiaire de synthèse subit plusieurs réactions successives et/ou simultanées dans son milieu réactionnel, en limitant les étapes de séparation et en supprimant les étapes de purification et de séchage des composés intermédiaires de synthèse.

L'expression « milieu réactionnel » désigne un milieu dans lequel ont lieu les réactions chimiques.

Par « isolement », on désigne la séparation d'un composé intermédiaire de synthèse du milieu réactionnel, éventuellement suivi de sa purification et/ou de son séchage. Les méthodes de séparation et/ou de purification d'un composé intermédiaire sont bien connues de l'homme du métier. Peuvent par exemple être citée la filtration, la chromatographie, la centrifugation, l'extraction par solvant, la distillation, etc .... Un lavage à l'eau ne sera pas considéré comme une méthode de séparation, ni comme une méthode de purification au sens de la présente invention et donc ne conduit pas à un isolement d'un intermédiaire de synthèse.

Dans le procédé de synthèse de l'invention la première étape, l'étape A1, permet d'obtenir un composé intermédiaire de formule (V) par une réaction de halogénométhylation d'un aldéhyde aromatique de formule (VI) en présence d'un agent de halogénométhylation selon le schéma réactionnel suivant : 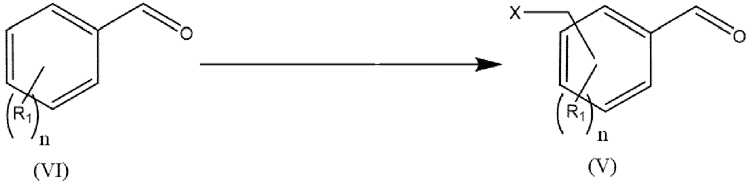 dans lequel :
- R₁ représente, indépendamment les uns des autres, un alkyle en C1-C6, de préférence un alkyle en C1-C3 ;
- n est un nombre entier égal à 0, 1, 2, 3 ou 4 ; et
- X est un atome d'halogène choisi dans le groupe constitué par le chlore, le brome, le fluor, l'iode ; plus préférentiellement choisi parmi un atome de chlore et un atome de brome ; plus préférentiellement encore est un atome de chlore.

Plus préférentiellement dans les composés de formule (VI) et (V), n est un entier égal à 3 ; R₁ représente, indépendamment les uns des autres, un alkyle en C1-C6, de préférence un alkyle en C1-C3 ; et X est un atome d'halogène choisi dans le groupe constitué par le chlore, le brome, le fluor et l'iode, plus préférentiellement choisi parmi un atome de chlore et un atome de brome, plus préférentiellement encore est un atome de chlore. Plus préférentiellement encore, n est un entier égal à 3 ; R₁ est un alkyle en C1-C3 ; et X est choisi parmi un atome de chlore ou un atome de brome, de préférence un atome de chlore. De manière encore plus préférée, n est égal à 3 ; les groupes R₁ sont identiques, situés en position ortho- et para- de la fonction aldéhyde et sont un alkyle en C1-C3, de préférence le méthyle, l'éthyle ou le propyle ; et X est choisi un atome de chlore ou un atome de brome, de préférence un atome de chlore.

L'étape A1 est une réaction d'halogénométhylation. Par « réaction d'halogénométhylation », on entend une réaction de type substitution électrophile, suivie d'une substitution nucléophile au cours de laquelle un composé aromatique est alkylé avec un agent d'halogénométhylation en présence ou non d'un catalyseur. Lorsque l'agent d'halogénométhylation comprend au moins un atome de chlore, on parlera alors de chlorométhylation. Lorsque l'agent d'halogénométhylation comprend au moins un atome de brome, on parlera alors de bromométhylation.

L'agent d'halogénométhylation peut être tout agent connu. Préférentiellement, l'agent de halogénométhylation est un donneur de formol dans une solution d'acide chlorhydrique ou d'acide bromhydrique en présence ou non d'un acide de Lewis tel que des halogénures de zinc ou d'un acide carboxylique tel que l'acide acétique. Plus préférentiellement encore, l'agent d'halogénométhylation est un mélange de donneur de formol et d'acide chlorhydrique.

Préférentiellement, dans le procédé de synthèse de l'invention, l'agent de halogénométhylation est préchauffé à une température T1 comprise dans un domaine allant de 23°C à 50°C, plus préférentiellement dans un domaine allant de 30°C à 45°C avant sa mise en contact avec le composé de formule (VI). La mise en contact de l'agent d'halogénométhylation avec le composé de formule (VI) s'effectue, préférentiellement, directement après son préchauffage.

Lorsque l'agent d'halogénométhylation est un donneur de formol, la quantité du donneur de formol dans l'étape A1 peut être comprise dans un domaine allant de 1 équivalent molaire à 7 équivalents molaires, de préférence de 1,2 équivalents molaires à 6 équivalents molaires, plus préférentiellement encore de 2 équivalents molaires à 6 équivalents molaires par rapport à la quantité de composé de formule (VI).

Préférentiellement, le donneur de formol peut être choisi parmi le paraformaldéhyde et le formaldéhyde. De préférence, le donner de formol est le paraformaldéhyde.

Préférentiellement, la solution d'acide chlorhydrique utilisée dans le cadre de la présente invention peut être une solution d'acide chlorhydrique à une concentration comprise dans un domaine allant de 15 % à 50 % en volume, plus préférentiellement de 27 % à 42 % en volume, plus préférentiellement de 30 % à 40 % en volume.

Préférentiellement, dans le procédé de synthèse de l'invention, le composé de formule (VI) peut être ajouté en une seule fois, notamment directement, après le préchauffage de l'agent de d'halogénométhylation.

Préférentiellement, l'étape A1 peut s'effectuer à une température T2 comprise dans un domaine allant de 60°C à 100°C, plus préférentiellement dans un domaine allant de 70°C à 90°C.

Selon un mode de réalisation, un solvant organique S1 peut être ajouté à la fin de l'étape A1 ; puis on peut effectuer une étape de récupération de la phase organique ; le solvant organique S1 pouvant être choisi parmi les solvants halogénés.

Par « solvant halogéné » ou « solvant organique halogéné », on entend un solvant comprenant au moins un hydrocarbure halogéné, c'est-à-dire un hydrocarbure comportant un ou plusieurs substituants halogènes. Préférentiellement, le solvant halogéné est un solvant chloré, c'est-à-dire un solvant comprenant au moins un hydrocarbure chloré, c'est-à-dire un hydrocarbure comportant un ou plusieurs substituants chlore. A titre de solvant chloré, le solvant organique S1 peut être choisi dans le groupe constitué par le dichlorométhane, le dichloroéthane, le chloroforme ; préférentiellement le solvant organique S1 peut être le dichlorométhane.

Préférentiellement, le composé intermédiaire de formule (V) n'est pas isolé, ni séché à l'issue de l'étape A1.

Le composé intermédiaire de formule (V) subit une réaction de substitution nucléophile avec un composé imidazole de formule (IV) (c'est l'étape A2) directement dans le même milieu réactionnel que celui de l'étape A1.

Ainsi le procédé de l'invention comprend une étape A2 d'obtention d'un composé intermédiaire de formule (III) par substitution nucléophile du composé intermédiaire de formule (V) précédent avec un composé imidazole de formule (IV) selon le schéma réactionnel suivant : 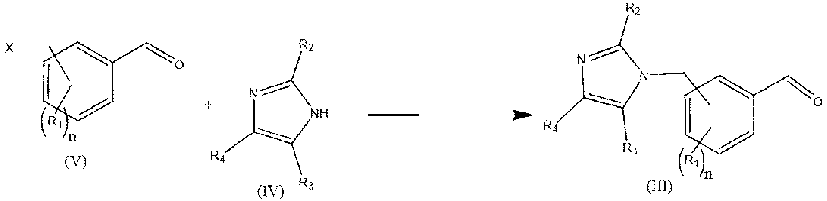 dans lequel :
- R₁ représente, indépendamment les uns des autres, un alkyle en C1-C6, de préférence un alkyle en C1-C3 ;
- n est un nombre entier égal à 0, 1, 2, 3 ou 4 ;
- X est un atome d'halogène choisi dans le groupe constitué par le chlore, le brome, le fluor, l'iode, plus préférentiellement choisi parmi un atome de chlore et un atome de brome, plus préférentiellement encore est un atome de chlore;
- R₂ représente un atome d'hydrogène, un alkyle en C1-C12, de préférence un alkyle en C1-C6, plus préférentiellement un alkyle en C1-C3 ; et
- R₃ et R₄, indépendamment l'un de l'autre, représente un atome d'hydrogène, un alkyle en C1-C12 (de préférence un alkyle en C1-C6, plus préférentiellement un alkyle en C1-C3), ou forment ensemble avec les atomes de carbone auxquels ils se rattachent un cycle, de préférence un cycle aromatique, plus préférentiellement le phényle.

Préférentiellement dans le composé de formule (V), n est un entier égal à 3 ; R₁ représente, indépendamment les uns des autres, un alkyle en C1-C6, de préférence un alkyle en C1-C3 ; et X est un atome d'halogène choisi dans le groupe constitué par le chlore, le brome, le fluor et l'iode, plus préférentiellement choisi parmi un atome de chlore et un atome de brome, plus préférentiellement encore est un atome de chlore. Plus préférentiellement encore, n est un entier égal à 3 ; R₁ est un alkyle en C1-C3 ; et X est choisi parmi un atome de chlore ou un atome de brome, de préférence un atome de chlore. De manière encore plus préférée, n est égal à 3 ; les groupes R₁ sont identiques, situés en position ortho- et para- de la fonction aldéhyde et sont un alkyle en C1-C3, de préférence le méthyle, l'éthyle ou le propyle ; et X est choisi un atome de chlore ou un atome de brome, de préférence un atome de chlore.

Préférentiellement dans le composé de formule (IV), R₂ représente un atome d'hydrogène ou un alkyle en C1-C6, plus préférentiellement un alkyle en C1-C3 ; et R₃ et R₄ représentent un atome d'hydrogène ou un alkyle en C1-C6, plus préférentiellement un alkyle en C1-C3 ou forment ensemble avec les atomes de carbone auxquels ils se rattachent un phényle. Plus préférentiellement encore dans le composé de formule (IV), R₂ représente un alkyle en C1-C3, de préférence le méthyle, l'éthyle ou le propyle ; et R₃ et R₄ représentent un atome d'hydrogène ou un alkyle en C1-C3 (de préférence le méthyle, l'éthyle, le propyle) ou forment ensemble avec les atomes de carbone auxquels ils se rattachent un phényle. Plus préférentiellement encore, R₂ représente un alkyle en C1-C3, de préférence le méthyle, l'éthyle ou le propyle ; et R₃ et R₄ sont identiques et sont un atome d'hydrogène.

Préférentiellement dans le composé de formule (III), n est un entier égal à 3 ; R₁ représente, indépendamment les uns des autres, un alkyle en C1-C6, de préférence un alkyle en C1-C3 ; R₂ représente un atome d'hydrogène ou un alkyle en C1-C6, plus préférentiellement un alkyle en C1-C3 ; et R₃ et R₄ représentent un atome d'hydrogène ou un alkyle en C1-C6, plus préférentiellement un alkyle en C1-C3 ou forment ensemble avec les atomes de carbone auxquels ils se rattachent un phényle. Plus préférentiellement encore, n est un entier égal à 3 ; R₁ est un alkyle en C1-C3 ; R₂ représente un alkyle en C1-C3, de préférence le méthyle, l'éthyle ou le propyle ; et R₃ et R₄ représentent un atome d'hydrogène ou un alkyle en C1-C3 (de de préférence le méthyle, l'éthyle ou le propyle) ou forment ensemble avec les atomes de carbone auxquels ils se rattachent un phényle. De manière encore plus préférée, n est égal à 3 ; les groupes R₁ sont identiques, situés en position ortho- et para- de la fonction aldéhyde et sont un alkyle en C1-C3, de préférence le méthyle, l'éthyle ou le propyle ; R₂ représente un alkyle en C1-C3, de préférence le méthyle, l'éthyle ou le propyle ; et R₃ et R₄ sont identiques et sont un atome d'hydrogène.

L'étape A2 peut s'effectuer en présence d'un solvant organique S2 choisi dans le groupe constitué par les solvants halogénés, le diméthylsulfoxyde, l'acétone, les alcools tels que l'isopropanol, les éthers tels que le tétrahydrofurane, l'acétate d'éthyle. Préférentiellement, le solvant organique S2 est un solvant halogéné, de préférence un solvant chloré, plus préférentiellement S2 est le dichlorométhane.

Préférentiellement, le solvant organique S2 est identique au solvant organique S1. Ainsi, lorsque le solvant organique S1 est un solvant halogéné, de préférence un solvant chloré, plus préférentiellement le dichlorométhane alors S2 est également un solvant halogéné, de préférence un solvant chloré, plus préférentiellement le dichlorométhane.

Préférentiellement, dans l'étape A2, la quantité de composé de formule (IV) peut être comprise dans un domaine allant de 1,1 équivalents molaires à 6 équivalents molaires, plus préférentiellement allant de 2 équivalents molaires à 4 équivalents molaires par rapport au composé de formule (V).

Préférentiellement, l'étape A2 s'effectue à une température inférieure ou égale à la température de reflux du solvant organique S2.

Préférentiellement, la quantité de solvant organique S2 est d'au moins 0,5 volume par rapport à 1 volume de solvant organique S1, plus préférentiellement est comprise dans un domaine allant de 1 volume à 10 volumes par rapport à 1 volume de solvant organique S1.

Le procédé de l'invention peut éventuellement comprendre, l'issue de l'étape A2 et avant l'étape A3, au moins une étape de lavage à l'eau. A l'issue de cette étape de lavage, on peut effectuer une étape de récupération de la phase organique et le procédé peut continuer directement avec la réalisation de l'étape A3.

Préférentiellement, le composé intermédiaire de formule (III) n'est pas isolé, et ne subit pas d'étapes supplémentaires de purification autre que des lavages à l'eau optionnels à l'issue de l'étape A2.

Le composé intermédiaire de formule (III) subit une réaction de condensation avec l'hydroxylamine (c'est l'étape A3) directement, notamment, dans le même milieu réactionnel que celui de l'étape A2.

Le procédé de synthèse de l'invention comprend une étape A3 d'obtention du composé de formule (I) par une réaction de condensation du composé intermédiaire de formule (III) précédent avec l'hydroxylamine (II) selon le schéma réactionnel suivant : 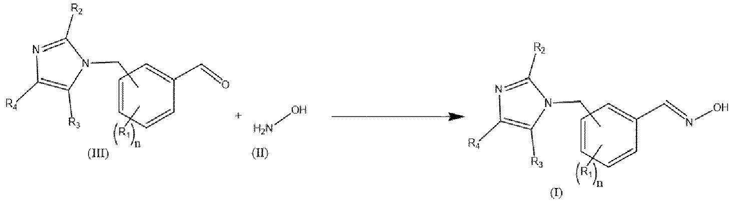 avec R₁, R₂, R₃, R₄ et n tels que définis ci-dessus y compris avec leurs modes préférés,

Préférentiellement dans le composé de formule (III) et dans le composé de formule (I), n est un entier égal à 3 ; R₁ représente, indépendamment les uns des autres, un alkyle en C1-C6, de préférence un alkyle en C1-C3 ; R₂ représente un atome d'hydrogène ou un alkyle en C1-C6, plus préférentiellement un alkyle en C1-C3 ; et R₃ et R₄ représentent un atome d'hydrogène ou un alkyle en C1-C6, plus préférentiellement un alkyle en C1-C3 ou forment ensemble avec les atomes de carbone auxquels ils se rattachent un phényle. Plus préférentiellement encore, n est un entier égal à 3 ; R₁ est un alkyle en C1-C3 ; R₂ représente un alkyle en C1-C3, de préférence le méthyle, l'éthyle ou le propyle ; et R₃ et R₄ représentent un atome d'hydrogène ou un alkyle en C1-C3 (de préférence le méthyle, l'éthyle ou le propyle) ou forment ensemble avec les atomes de carbone auxquels ils se rattachent un phényle. De manière encore plus préférée, n est égal à 3 ; les groupes R₁ sont identiques, situés en position ortho- et para- de la fonction aldéhyde, respectivement la fonction oxime, et sont un alkyle en C1-C3, de préférence le méthyle, l'éthyle ou le propyle ; R₂ représente un alkyle en C1-C3, de préférence le méthyle, l'éthyle ou le propyle ; et R₃ et R₄ sont identiques et sont un atome d'hydrogène.

Préférentiellement, l'étape A3 s'effectue dans un solvant organique S3 choisi dans le groupe constitué par les solvants halogénés, l'acétone, les alcools tels que l'isopropanol, les éthers tels que le tétrahydrofurane, l'acétate d'éthyle. Préférentiellement, le solvant organique S3 est un solvant organique halogéné, de préférence un solvant organique chloré, plus préférentiellement le solvant organique S3 est le dichlorométhane.

Préférentiellement, le composé de formule (II) est ajouté dans le réacteur où se déroule l'étape A3 sous la forme d'une solution aqueuse ou bien sous la forme d'un sel. Préférentiellement l'hydroxylamine est ajoutée sous forme de sel.

Préférentiellement, l'hydroxylamine sous forme de sel est choisie dans le groupe formé par les sels de sulfate d'hydroxylamine, les sels de chlorure d'hydroxylamine et les mélanges de ces sels. Dans le cas de l'utilisation de l'hydroxylamine sous forme de sel, une base pourra préférentiellement être additionnée au milieu réactionnel. À titre d'exemple de base, on peut citer l'acétate de sodium ou la triéthylamine. La quantité de base additionnée sera comprise dans un domaine allant de 1 à 2 équivalents molaires par rapport l'hydroxylamine générée, préférentiellement de 1 à 1,2 équivalents molaires par rapport l'hydroxylamine générée. Par « hydroxylamine générée », on entend le cation (NH3⁺OH) du sel d'hydroxylamine qui est libéré lors de la mise en contact dudit sel avec l'eau. Lorsqu'on utilise une base, on mélange ladite base avec le sel d'hydroxylamine, puis on dissout l'ensemble dans l'eau.

Préférentiellement, le solvant organique S3 est identique au solvant organique S2. Ainsi lorsque le solvant organique S2 est un solvant halogéné, de préférence un solvant chloré, plus préférentiellement le dichlorométhane alors S3 est également un solvant halogéné, de préférence un solvant chloré, plus préférentiellement le dichlorométhane.

Préférentiellement, la réaction A3 s'effectue en présence d'un co-solvant, ledit co-solvant étant choisi dans le groupe constitué par les alcools tels que l'éthanol ou l'isopropanol. Plus préférentiellement encore, le solvant organique S3 est un solvant halogéné et le co-solvant est un alcool, plus préférentiellement le solvant organique S3 est solvant chloré et le co-solvant est un alcool. Plus préférentiellement encore, le solvant organique S3 est le dichlorométhane et le co-solvant est un alcool, de préférence l'isopropanol ou l'éthanol.

Le procédé de synthèse de l'invention peut comprendre une étape optionnelle A4 d'isolement, de purification et de séchage du composé de formule (I) afin de récupérer le composé de formule (I).

Préférentiellement, parmi les composés de formule (I) qui sont synthétisés selon le procédé de l'invention décrit ci-dessus, le composé de formule (la) est plus particulièrement préféré

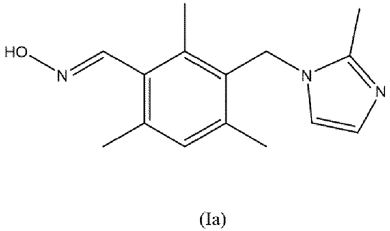

Un second objet de la présente invention concerne un procédé de synthèse d'un composé de formule (X), ledit procédé comprenant les étapes suivantes :
- une étape (i) de synthèse du composé de formule (I) par le procédé de synthèse tel que décrit ci-dessus,
- une étape (ii) de transformation du composé de formule (I) en composé de formule (X) en présence d'au moins un solvant organique S4 et d'au moins un oxydant selon le schéma réactionnel suivant : 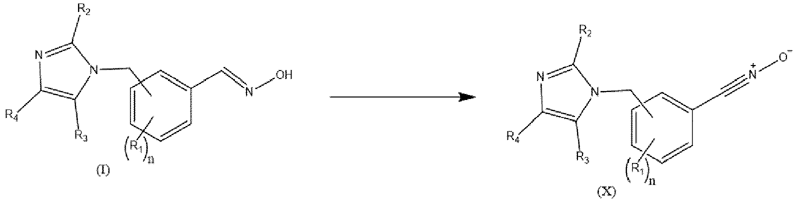 avec :
   - R₁ représente, indépendamment les uns des autres, un alkyle en C1-C6, de préférence un alkyle en C1-C3 ;
   - R₂ représente un atome d'hydrogène ou un alkyle en C1-C12, de préférence un alkyle en C1-C6, plus préférentiellement un alkyle en C1-C3 ;
   - R₃ et R₄, indépendamment l'un de l'autre, représente un atome d'hydrogène, un alkyle en C1-C12, de préférence un alkyle en C1-C6, plus préférentiellement un alkyle en C1-C3, ou forment ensemble avec les atomes de carbone auxquels ils se rattachent un cycle, de préférence un cycle aromatique, plus préférentiellement le phényle ; et
   - n un nombre entier égal à 0, 1, 2, 3 ou 4 ; et
      - une étape (iii) de récupération du composé de formule (X).

Préférentiellement dans le composé de formule (I) et dans le composé de formule (III), n est un entier égal à 3 ; R₁ représente, indépendamment les uns des autres, un alkyle en C1-C6, de préférence un alkyle en C1-C3 ; R₂ représente un atome d'hydrogène ou un alkyle en C1-C6, plus préférentiellement un alkyle en C1-C3 ; et R₃ et R₄ représentent un atome d'hydrogène ou un alkyle en C1-C6, plus préférentiellement un alkyle en C1-C3, ou forment ensemble avec les atomes de carbone auxquels ils se rattachent un phényle. Plus préférentiellement encore, n est un entier égal à 3 ; R₁ est un alkyle en C1-C3 ; R₂ représente un alkyle en C1-C3, de préférence le méthyle, l'éthyle ou le propyle ; et R₃ et R₄ représentent un atome d'hydrogène ou un alkyle en C1-C3 (de préférence le méthyle, l'éthyle ou le propyle) ou forment ensemble avec les atomes de carbone auxquels ils se rattachent un phényle. De manière encore plus préférée, n est égal à 3 ; les groupes R₁ sont identiques, situés en position ortho-et para- de la fonction oxime , respectivement la fonction oxide de nitrile, et sont un alkyle en C1-C3, de préférence le méthyle, l'éthyle ou le propyle ; R₂ représente un alkyle en C1-C3, de préférence le méthyle, l'éthyle ou le propyle ; et R₃ et R₄ sont identiques et sont un atome d'hydrogène.

L'étape (i) ainsi que ces modes préférés ont été décrits ci-dessus.

Préférentiellement dans l'étape (ii), l'oxydant est choisi dans le groupe constitué par l'hypochlorite de sodium, le N-bromosuccinimide en présence d'une base, le N-chlorosuccinimide en présence d'une base, préférentiellement l'oxydant est l'hypochlorite de sodium.

Préférentiellement, le solvant organique S4 est choisi dans le groupe formé par les alcools tels que l'éthanol ou l'isopropanol, les solvants chlorés tels que le chloroforme ou le dichlorométhane, l'acétate d'éthyle.

Avantageusement, la quantité d'agent oxydant est de 1 à 5 équivalents molaires, préférentiellement de 1 à 2 équivalents molaires, par rapport à la quantité molaire d'oxime de formule (I).

Préférentiellement, parmi les composés de formule (X) qui sont synthétisés selon le procédé de l'invention décrit ci-dessus, le composé de formule (Xa) est plus particulièrement préféré

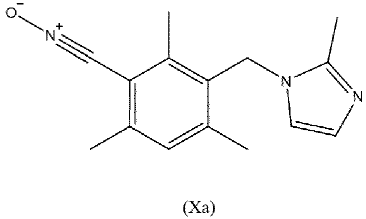

Les exemples figurant ci-après sont présentés à titre illustratif et ne sont pas limitatifs de l'invention.

### EXEMPLES

### Essai 1 :

1.1-Synthèse du composé de formule (la) oxime de 2,4,6-triméthyl-3-((2-méthyl-1H-imidazol-1-yl)méthyl)benzaldéhyde selon le procédé de l'invention (procédé sans isolement des intermédiaires de synthèse B et C))

Le composé de formule (I) est synthétisé selon le schéma réactionnel suivant :

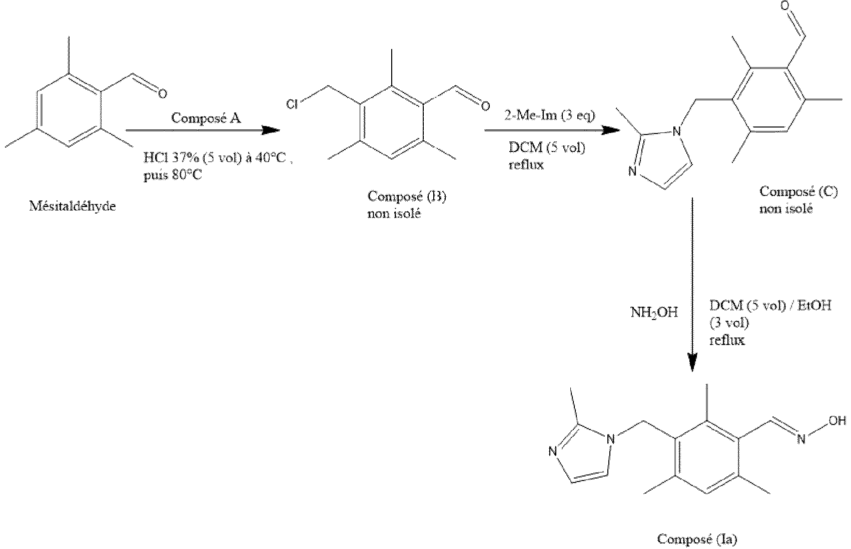

On mélange dans un réacteur de 25 L à 40°C, 5,58 L d'une solution d'acide chlorhydrique (37% en solution dans l'eau) et 0,57 kg de paraformaldéhyde (composé A, 18,82 mol, 2.5 eq. (eq. = équivalent molaire)). Après 10 min d'agitation à 40°C, le mélange devient homogène et on ajoute 1,12 kg de mésitaldéhyde (7,53 mol). Le mélange est maintenu sous agitation et chauffé à 80°C progressivement c'est-à-dire 2°C/min. Après 4 heures d'agitation à 80°C, le mélange est refroidi à 40°C et dilué par du dichlorométhane (1,12 L). La phase organique est séparée et utilisée dans l'étape suivante sans purification supplémentaire.

1,75 kg d'une solution de 2-méthyl- 1H-imidazole, ((2-Me-Im), 21.3 mol) dans du dichlorométhane (5.92 L) est portée à reflux pour atteindre une solubilisation partielle, puis la solution préparée précédemment de 3-(chlorométhyl)-2,4,6-trimethylbenzaldehyde (composé B) dans le dichlorométhane (7,53 mol dans 1,12 L de dichlorométhane) est ajoutée à la solution de 2-méthyl- 1H-imidazole. On obtient un mélange orangé qui est maintenu à reflux sous agitation pendant 2 heures. Le chauffage est stoppé et le mélange réactionnel est lavé 5 fois avec 5,25 L d'eau. La phase organique est séparée et utilisée dans l'étape suivante sans traitement supplémentaire (c'est-à-dire sans purification et sans isolement).

Dans un réacteur de 50 L, on ajoute, à la solution précédemment obtenue de 2,4,6-trimethyl-3-[(2-methyl-1H-imidazol-1-yl)méthyl]benzaldéhyde dans le dichlorométhane, 4.38 L de l'éthanol et 1,81 L de dichlorométhane ; puis on ajoute 0,35 L d'une solution aqueuse d'hydroxylamine à 50 % en masse dans l'eau (6 mol). Le mélange est chauffé pour être porté à reflux et est maintenu sous agitation pendant 7 heures. La suspension est ensuite refroidie pour atteindre la température ambiante de 23°C, puis concentrée et filtrée. Le solide ainsi obtenu est ensuite lavé sur filtre par l'addition de 0,58 L d'éthanol puis séché sous vide pendant un jour. Après séchage sous vide, un solide blanc est récupéré avec un rendement global sur les 3 étapes de 65 % (1.26 Kg, 4.9 mol).

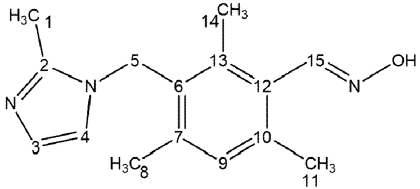

**[Table 1]**

| N° | δ¹H (ppm) | δ¹³ C (ppm) |
|---|---|---|
| 1 | 2,31 | 12,7 |
| 2 | (-) | 143,4 |
| 3 | 6,58 | 125,8 |
| 4 | 6,22 | 116,9 |
| 5 | 4,97 | 43,2 |
| 6 | (-) | 129,3 |
| 7 | (-) | 136,2 |
| 8 | 2,23 | 20,2 |
| 9 | 6,97 | 130 |
| 10 | (-) | 137,3 |
| 11 | 2,15 | 19,1 |
| 12 | (-) | 129,1 |
| 13 | (-) | 136,1 |
| 14 | 2,11 | 15,9 |
| 15 | 8,25 | 147,4 |
| OH | 11,11 | (-) |

### 1.2- Synthèse du composé de formule (I) oxime de 2,4,6-triméthyl-3-((2-méthyl-1H-imidazol-1-yl)méthyl)benzaldéhyde selon un procédé de l'art antérieur décrit dans les exemples de WO2015059269

L'oxime de 2,4,6-triméthyl-3-((2-méthyl-1H-imidazol-1-yl)méthyl)benzaldéhyde est synthétisée selon un procédé de l'art antérieur selon le schéma réactionnel ci-dessous.

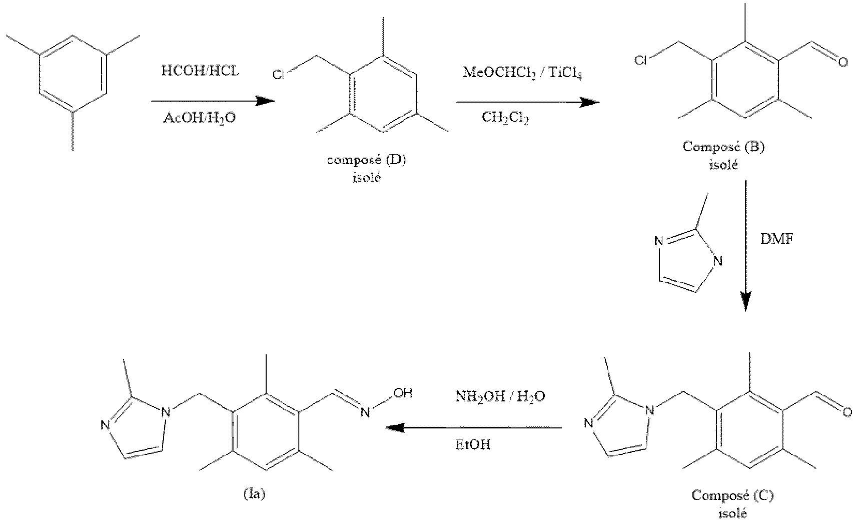

### 1.2.1- Synthèse du 2-(chlorométhyl)1,3,5-triméthylbenzène (composé D)

Ce composé peut être obtenu selon une procédure décrite dans l'article suivant : Zenkevich, I. G.; Makarov, A. A.; Russian Journal of General Chemistry; vol. 77; nb. 4; (2007); p. 611 -619 (Zhurnal Obshchei Khimii; vol. 77; nb. 4; (2007); p. 653 - 662)

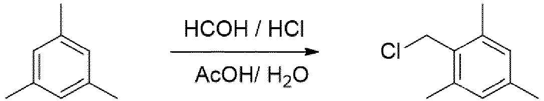

Un mélange de mésitylène (100,0 g, 0,832 mol), de para-formaldéhyde (26,2 g, 0,874 mol) et d'acide chlorhydrique (240 ml, 37 %, 2,906 mol) dans l'acide acétique (240 ml) est agité et chauffé très lentement (1,5 heures) jusqu'à 37°C. Après retour à température ambiante, le mélange est dilué par de l'eau (1,0 l) avec CH₂Cl₂ (200 ml), le produit est extrait par CH₂Cl₂ (4 fois par 50 ml). Les phases organiques sont rassemblées, puis lavées par l'eau (5 fois par 100 ml) et évaporées jusqu'à 11-12 mbar (Température du bain = 42°C). Une huile incolore (133,52 g, rendement 95 %) est obtenue. Après 15-18 heures à +4°C, l'huile a cristallisé. Les cristaux sont filtrés, lavés par de l'éther de pétrole refroidi à -18°C (40 ml), puis séchés pendant 3 à 5 heures sous pression atmosphérique à température ambiante. Un solide blanc (95,9 g, rendement 68 %) de point de fusion 39°C est obtenu. La pureté molaire est supérieure à 96 % (RMN ¹H).

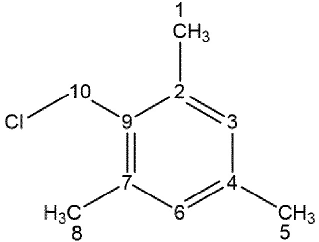

**[Table 2]**

| N° | δ¹H (ppm) | δ ¹³C (ppm) |
|---|---|---|
| 1 | 2,27 | 18,4 |
| 2 | (-) | 136,9 |
| 3 | 6,81 | 128,5 |
| 4 | (-) | 137,4 |
| 5 | 2,15 | 20,3 |
| 6 | 6,81 | 128,5 |
| 7 | (-) | 136,9 |
| 8 | 2,27 | 18,4 |
| 9 | (-) | 130,5 |
| 10 | 4,69 | 41,3 |

### 1.2.2- Synthèse du 3-(chlorométhyl)-2,4,6-triméthylbenzaldéhyde (composé B)

Ce composé peut être obtenu selon une procédure décrite dans l'article suivant : Yakubov, A. P.; Tsyganov, D. V.; Belen'kii, L. I.; Krayushkin, M. M.; Bulletin of the Academy of Sciences of the USSR, Division of Chemical Science (English Translation); vol. 40; nb. 7.2; (1991); p. 1427 - 1432 (Izvestiya Akademii Nauk SSSR, Seriya Khimicheskaya; nb. 7; (1991); p. 1609 - 1615)

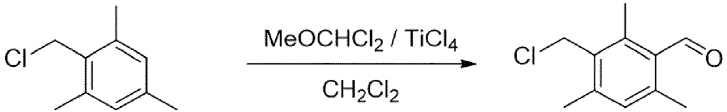

A une solution de TiCl₄ (90,0 g, 0,474 mol) dans le dichlorométhane (200 ml) à 17°C est ajoutée sous argon pendant 10-12 minutes une solution du 2-(chlorométhyl)-1,3,5-triméthylbenzène (20,0 g, 0,118 mol) et de dichlorométhylméthyl éther (27,26 g, 0,237 mol) dans le dichlorométhane (200 ml). Après agitation pendant 15-20 minutes à 17-20°C, de l'eau (1000 ml) et de la glace (500 g) sont ajoutées au milieu réactionnel. Après 10-15 minutes d'agitation, la phase organique est séparée. La phase aqueuse est extraite par CH₂Cl₂ (3 fois par 75 ml). Les phases organiques rassemblées sont lavées par l'eau (4 fois par 100 ml) et évaporées sous pression réduite pour conduire à un solide (Température du bain = 28°C). Le produit cible (22,74 g) est obtenu avec un rendement de 97 %. Son point de fusion est de 58 °C. La pureté molaire estimée par RMN ¹H est de 95%mol.

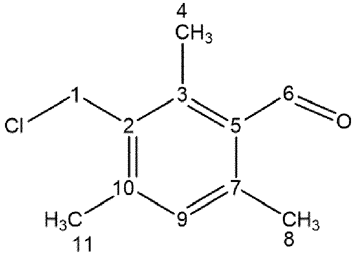

**[Table 3]**

| N° | δ¹H (ppm) | δ¹³C (ppm) |
|---|---|---|
| 1 | 4,77 | 40,6 |
| 2 | (-) | 132.9 |
| 3 | (-) | 139,5 |
| 4 | 2,51 | 14,4 |
| 5 | (-) | 131,4 |
| 6 | 10,43 | 194,2 |
| 7 | (-) | 140,1 |
| 8 | 2,41 | 19,3 |
| 9 | 6,99 | 131,2 |
| 10 | (-) | 142,4 |
| 11 | 2,34 | 19,8 |

### 1.2.3 - Synthèse du 2,4,6-triméthyl-3-((2-méthyl-1H-imidazol-1-yl)méthyl) benzaldéhyde (composé C) :

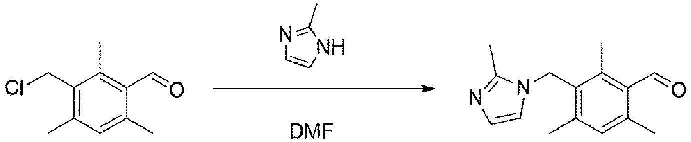

Un mélange de 3-(chlorométhyl)-2,4,6-triméthylbenzaldéhyde (10,0 g, 0,051 mol) et d'imidazole (10,44 g, 0,127 mol) dans le DMF (10 ml) est agité à 80°C pendant une heure.

Après retour à 40-50°C, le mélange est dilué par l'eau (200ml), et agité pendant 10 minutes. Le précipité obtenu est filtré et lavé sur le filtre par l'eau (4 fois par 25 ml) puis séché à température ambiante. Un solide blanc (7,92 g, rendement 64 %) de point de fusion de 161 °C est obtenu. La pureté molaire est 91 % (RMN ¹H).

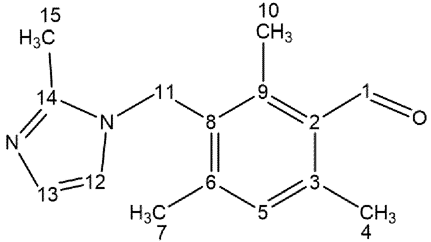

**[Table 4]**

| N° | δ¹H (ppm) | δ¹³C (ppm) |
|---|---|---|
| 1 | 10,45 | 194,2 |
| 2 | (-) | 131,5 |
| 3 | (-) | 139,5 |
| 4 | 2,44 | 19,6 |
| 5 | 7,04 | 131,2 |
| 6 | (-) | 142,5 |
| 7 | 2,19 | 19,5 |
| 8 | (-) | 131 |
| 9 | (-) | 139,5 |
| 10 | 2,34 | 14,6 |
| 11 | 5,02 | 42,5 |
| 12 | 6,24 | 116,9 |
| 13 | 6,59 | 125,9 |
| 14 | (-) | 143,5 |
| 15 | 2,32 | 12,7 |

### 1.2.4-Synthèse du 2,4,6-triméthyl-3-((2-méthyl-1H-imidazol-1-yl)méthyl)benzaldéhyde oxime (composé la) :

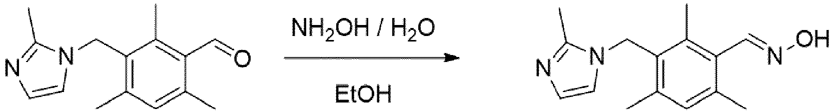

A une solution de 2,4,6-triméthyl-3-((2-méthyl-1H-imidazol-1-yl)méthyl) benzaldéhyde (20.3 g, 0,084mol) dans EtOH (110 ml) à 40°C, est ajoutée une solution aqueuse d'hydroxylamine (809 g, 0,134 mol, 50 % dans l'eau, Aldrich) dans EtOH (10 ml). Le milieu réactionnel est agité pendant 2,5 heures à une température de 50 à 55°C. Après retour à 23°C, le précipité obtenu est filtré et lavé deux fois sur le filtre par un mélange EtOH/H₂O (10 ml/15 ml) et séché pendant 15 à 20 heures sous pression atmosphérique à température ambiante. Un solide blanc (19.57 g, rendement 91 %) de point de fusion de 247°C est obtenu. La pureté molaire est supérieure à 87 % (RMN ¹H).

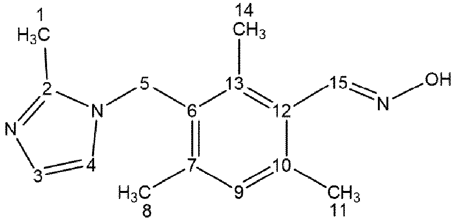

**[Table 5]**

| N° | δ¹H (ppm) | δ¹³C (ppm) |
|---|---|---|
| 1 | 2,31 | 12,7 |
| 2 | (-) | 143,4 |
| 3 | 6,58 | 125,8 |
| 4 | 6,22 | 116,9 |
| 5 | 4,97 | 43,2 |
| 6 | (-) | 129,3 |
| 7 | (-) | 136,2 |
| 8 | 2,23 | 20,2 |
| 9 | 6,97 | 130 |
| 10 | (-) | 137,3 |
| 11 | 2,15 | 19,1 |
| 12 | (-) | 129,1 |
| 13 | (-) | 136,1 |
| 14 | 2,11 | 15,9 |
| 15 | 8,25 | 147,4 |
| OH | 11,11 | (-) |

### 1.3- Comparaison du procédé de l'invention et du procédé de l'art antérieur

**[Table 6]**

| | | Synthèse du composé (la) selon le procédé de l'art antérieur (procédé 1.2) | Synthèse du composé (la) selon le procédé de l'invention (procédé 1.1) |
|---|---|---|---|
| Nombre d'intermédiaire isolé | | 3 | 0 |
| Rendement global | | 38 % | 65 % |
| Nombre de solvants utilisés | | 4 | 2 |
| Litres de solvant utilisés/kg de produit final | dichlorométhane | 71,4 | 7 |
| | alcool (éthanol, isopropanol) | 7,2 | 3,9 |
| | éther de pétrole | 4,4 | 0 |
| | DMF | 1,3 | 0 |
| Litres dérivés aqueux (eau, HCl) utilisés/kg de produit final | | 190,2 | 25,5 |

De manière totalement inattendue, le procédé de synthèse selon l'invention du composé de formule (la) permet une réduction du nombre d'intermédiaire isolé tout en améliorant le rendement du procédé. Il permet également une diminution du nombre de solvants utilisés, donc une simplification de manipulation par un opérateur et une amélioration du temps de cycle global en supprimant les temps liés aux étapes de purification et de séchage des intermédiaires. Enfin il permet une diminution des effluents et donc également de leurs traitements.

### Essai 2 :

2.1 - Procédé de synthèse du composé de formule (Xa) à partir du composé de formule (la) synthétisé selon le procédé de l'invention (conforme à l'invention)

Le composé de formule (Xa) est obtenu à partir du composé de formule (la) obtenu par le procédé sans isolement des intermédiaires de l'invention et selon le schéma réactionnel suivant :

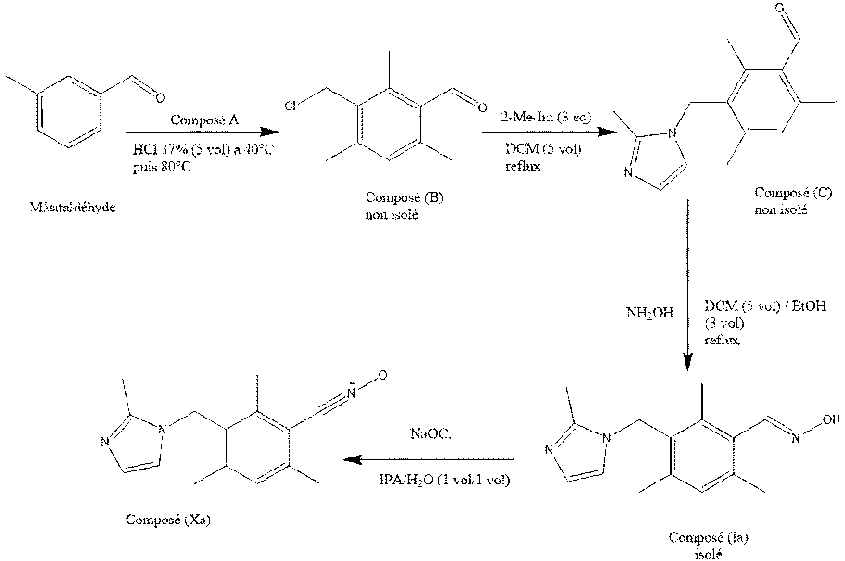

1,26 Kg de l'oxime 2,4,6-triméthyl-3-((2-méthyl-1H-imidazol-1-yl)méthyl)benzaldehyde (4.9 mol), composé (la) obtenu selon le procédé sans isolement des intermédiaires de l'invention tel que décrit au paragraphe 1.1 ci-dessus est mis en suspension dans l'isopropanol/eau (1,3 l/1,3 l). Le mélange est refroidi à 0°C et 6,44 L d'une solution d'hypochlorite de sodium à 14 % dans l'eau (14.7 mol) est ajoutée sur une période de 30 min. Après la fin de l'addition, le mélange est maintenu sous agitation à 2-3°C pendant 2 heures.

Le produit est ensuite filtré et la poudre beige est remis en suspension dans l'eau (4,73l) et agitée pendant 1 h puis filtrée. L'opération est renouvelée 4 fois. Après la dernière filtration, la poudre est broyée et séchée sous vide (40 mbar) pendant 8 h à 50°C puis une nuit à 40°C sous vide (50 mbar). Le N-oxyde de 2,4,6-triméthyl-3-((2-méthyl-1H-imidazol-1-yl)méthyl)benzonitrile est obtenu avec un rendement de 80 % (1 kg, 3.92 mol,) sous forme d'un solide légèrement jaune.

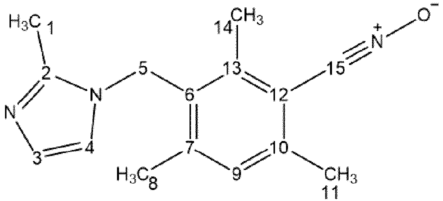

**[Table 7]**

| N° | δ¹H (ppm) | δ¹³C (ppm) |
|---|---|---|
| 1 | 2,3 | 12,6 |
| 2 | (-) | 143,6 |
| 3 | 6,59 | 126,1 |
| 4 | 6,27 | 117,1 |
| 5 | 4,99 | 43 |
| 6 | (-) | 130,6 |
| 7 | (-) | 140,7 |
| 8 | 2,16 | 19,2 |
| 9 | 7,12 | 129,9 |
| 10 | (-) | 141 |
| 11 | 2,34 | 20 |
| 12 | (-) | 112,1 |
| 13 | (-) | 140,8 |
| 14 | 2,28 | 17,7 |
| 15 | (-) | NI |

### 2.2 - Synthèse du composé de formule (Xa) selon un procédé de l'art antérieur (non conforme à l'invention)

Le N-oxyde de 2,4,6-triméthyl-3-2((2-méthyl-1H-imidazol-1-yl)benzonitrile (composé Xa) est préparé selon le schéma réactionnel suivant :

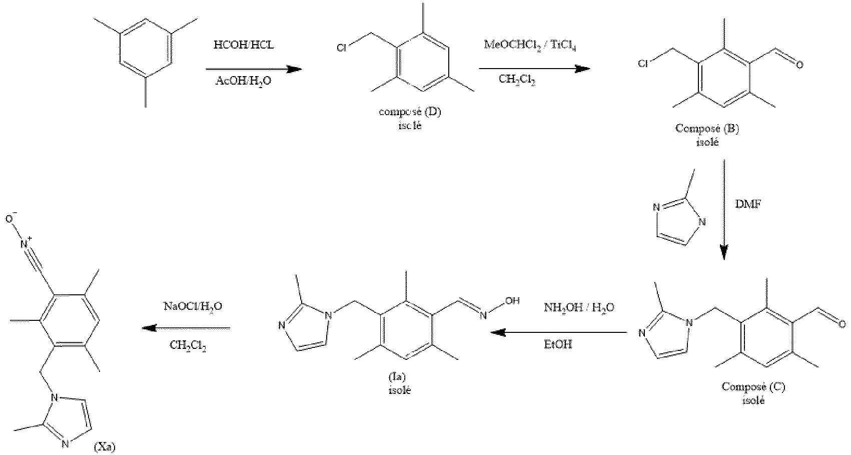

### 2.2.1 Synthèse du 2-(chlorométhyl)1,3,5-triméthylbenzène (composé (D))

Ce composé peut être obtenu selon une procédure décrite dans l'article suivant : Zenkevich, I. G.; Makarov, A. A.; Russian Journal of General Chemistry; vol. 77; nb. 4; (2007); p. 611 -619 (Zhurnal Obshchei Khimii; vol. 77; nb. 4; (2007); p. 653 - 662)

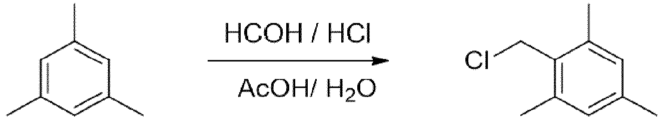

Un mélange de mésitylène (100,0 g, 0,832 mol), de para-formaldéhyde (26,2 g, 0,874 mol) et d'acide chlorhydrique (240 ml, 37 %, 2,906 mol) dans l'acide acétique (240 ml) est agité et chauffé très lentement (1,5 heures) jusqu'à 37°C. Après retour à température ambiante, le mélange est dilué par de l'eau (1,0 l) avec CH2Cl2 (200 ml), le produit est extrait par CH2Cl2 (4 fois par 50 mL). Les phases organiques sont rassemblées, puis lavées par l'eau (5 fois par 100 ml) et évaporées jusqu'à 11-12 mbar (Température du bain = 42°C). Une huile incolore (133,52 g, rendement 95 %) est obtenue. Après 15-18 heures à +4°C, l'huile a cristallisé. Les cristaux sont filtrés, lavés par de l'éther de pétrole refroidi à -18°C (40 ml), puis séchés pendant 3 à 5 heures sous pression atmosphérique à température ambiante. Un solide blanc (95,9 g, rendement 68 %) de point de fusion 39 °C est obtenu. La pureté molaire est supérieure à 96 % (RMN ¹H).

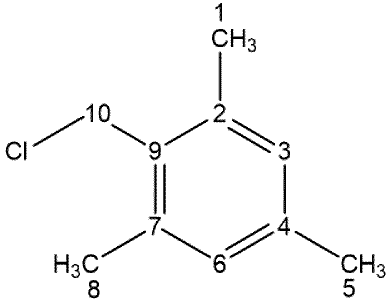

**[Table 8]**

| N° | δ¹H (ppm) | δ¹³C (ppm) |
|---|---|---|
| 1 | 2,27 | 18,4 |
| 2 | (-) | 136,9 |
| 3 | 6,81 | 128,5 |
| 4 | (-) | 137,4 |
| 5 | 2,15 | 20,3 |
| 6 | 6,81 | 128,5 |
| 7 | (-) | 136,9 |
| 8 | 2,27 | 18,4 |
| 9 | (-) | 130,5 |
| 10 | 4,69 | 41,3 |

### 2.2.2 - Synthèse du 3-(chlorométhyl)-2,4,6-triméthylbenzaldéhyde (Composé (B))

Ce composé peut être obtenu selon une procédure décrite dans l'article suivant : Yakubov, A. P.; Tsyganov, D. V.; Belen'kii, L. I.; Krayushkin, M. M.; Bulletin of the Academy of Sciences of the USSR, Division of Chemical Science (English Translation); vol. 40; nb. 7.2; (1991); p. 1427 - 1432 (Izvestiya Akademii Nauk SSSR, Seriya Khimicheskaya; nb. 7; (1991); p. 1609 - 1615)]

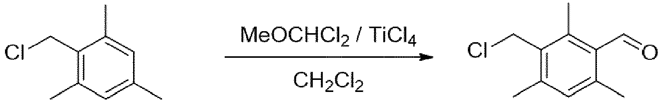

A une solution de TiCl₄ (90,0 g, 0,474 mol) dans le dichlorométhane (200 ml) à 17°C est ajoutée sous argon pendant 10-12 minutes une solution du 2-(chlorométhyl)-1,3,5-triméthylbenzène (20,0 g, 0,118 mol) et de dichlorométhylméthyl éther (27,26 g, 0,237 mol) dans le dichlorométhane (200 ml). Après agitation pendant 15-20 minutes à 17-20 °C, de l'eau (1000 ml) et de la glace (500 g) sont ajoutées au milieu réactionnel. Après 10-15 minutes d'agitation, la phase organique est séparée. La phase aqueuse est extraite par CH₂Cl₂ (3 fois par 75 ml). Les phases organiques rassemblées sont lavées par l'eau (4 fois par 100 ml) et évaporées sous pression réduite pour conduire à un solide (Température du bain = 28°C). Le produit cible (22,74 g) est obtenu avec un rendement de 97 %. Son point de fusion est de 58 °C. La pureté molaire estimée par RMN ¹H est de 95%mol.

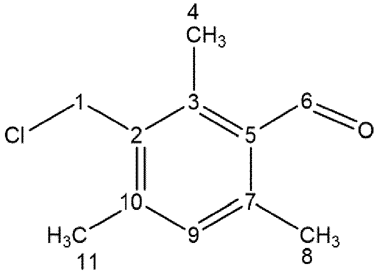

**[Table 9]**

| N° | δ¹H (ppm) | δ¹³C (ppm) |
|---|---|---|
| 1 | 4,77 | 40,6 |
| 2 | (-) | 132.9 |
| 3 | (-) | 139,5 |
| 4 | 2,51 | 14,4 |
| 5 | (-) | 131,4 |
| 6 | 10,43 | 194,2 |
| 7 | (-) | 140,1 |
| 8 | 2,41 | 19,3 |
| 9 | 6,99 | 131,2 |
| 10 | (-) | 142,4 |
| 11 | 2,34 | 19,8 |

### 2.2.3 - Synthèse du 2,4,6-triméthyl-3-((2-méthyl-1H-imidazol-1-yl)méthyl) benzaldéhyde (Composé (C))

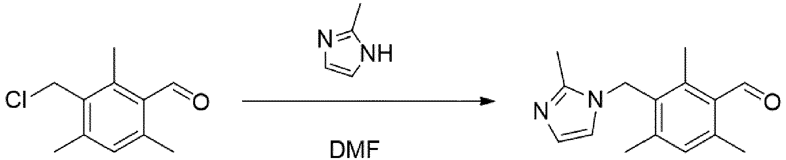

Un mélange de 3-(chlorométhyl)-2,4,6-triméthylbenzaldéhyde (10,0 g, 0,051 mol) et d'imidazole (10,44 g, 0,127 mol) dans le DMF (10 ml) est agité à 80°C pendant une heure.

Après retour à 40-50°C, le mélange est dilué par l'eau (200ml), et agité pendant 10 minutes. Le précipité obtenu est filtré et lavé sur le filtre par l'eau (4 fois par 25 ml) puis séché à température ambiante. Un solide blanc (7,92 g, rendement 64 %) de point de fusion de 161°C est obtenu. La pureté molaire est 91 % (RMN ¹H).

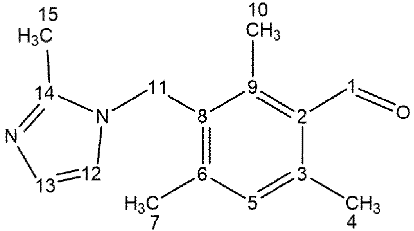

**[Table 10]**

| N° | δ¹H (ppm) | δ¹³C (ppm) |
|---|---|---|
| 1 | 10,45 | 194,2 |
| 2 | (-) | 131,5 |
| 3 | (-) | 139,5 |
| 4 | 2,44 | 19,6 |
| 5 | 7,04 | 131,2 |
| 6 | (-) | 142,5 |
| 7 | 2,19 | 19,5 |
| 8 | (-) | 131 |
| 9 | (-) | 139,5 |
| 10 | 2,34 | 14,6 |
| 11 | 5,02 | 42,5 |
| 12 | 6,24 | 116,9 |
| 13 | 6,59 | 125,9 |
| 14 | (-) | 143,5 |
| 15 | 2,32 | 12,7 |

### 2.2.4 - Synthèse du 2,4,6-triméthyl-3-((2-méthyl-1H-imidazol-1-yl)méthyl)benzaldéhyde oxime (composé (la))

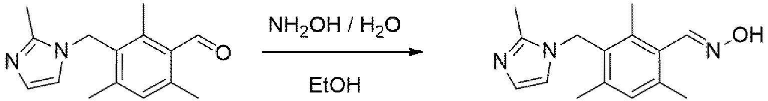

A une solution de 2,4,6-triméthyl-3-((2-méthyl-1H-imidazol-1-yl)méthyl) benzaldéhyde (20.3 g, 0,084mol) dans EtOH (110 ml) à 40°C, est ajoutée une solution aqueuse d'hydroxylamine (809 g, 0,134 mol, 50 % dans l'eau, Aldrich) dans EtOH (10 ml). Le milieu réactionnel est agité pendant 2,5 heures à une température de 50 à 55°C. Après retour à 23°C, le précipité obtenu est filtré et lavé deux fois sur le filtre par un mélange EtOH/H₂O (10 ml/15 ml) et séché pendant 15 à 20 heures sous pression atmosphérique à température ambiante. Un solide blanc (19.57 g, rendement 91 %) de point de fusion de 247°C est obtenu. La pureté molaire est supérieure à 87 % (RMN ¹H).

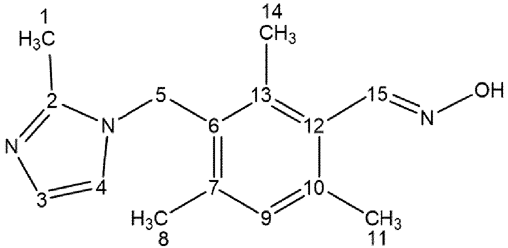

**[Table 11]**

| N° | δ¹H (ppm) | δ¹³C (ppm) |
|---|---|---|
| 1 | 2,31 | 12,7 |
| 2 | (-) | 143,4 |
| 3 | 6,58 | 125,8 |
| 4 | 6,22 | 116,9 |
| 5 | 4,97 | 43,2 |
| 6 | (-) | 129,3 |
| 7 | (-) | 136,2 |
| 8 | 2,23 | 20,2 |
| 9 | 6,97 | 130 |
| 10 | (-) | 137,3 |
| 11 | 2,15 | 19,1 |
| 12 | (-) | 129,1 |
| 13 | (-) | 136,1 |
| 14 | 2,11 | 15,9 |
| 15 | 8,25 | 147,4 |
| OH | 11,11 | (-) |

### 2.2.5 - Synthèse du 2,4,6-triméthyl-3-((2-méthyl-1H-imidazol-1-yl)méthyl)benzonitrile oxyde (composé (Xa))

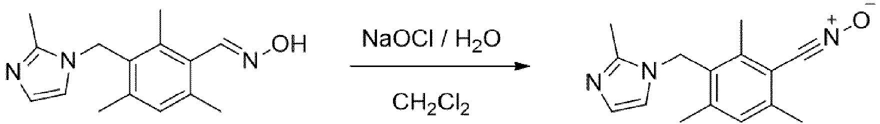

A un mélange de 2,4,6-triméthyl-3-((2-méthyl-1H-imidazol-1-yl)méthyl)benzaldéhyde oxime (8,80 g, 0,034 mol) dans CH₂Cl₂ (280 ml) à 6°C est ajoutée goutte à goutte une solution aqueuse de NaOCl (4 % de chlore actif, Aldrich, 49 ml) pendant 5 minutes. La température du milieu réactionnel est maintenue entre 6 et 8°C. Le milieu réactionnel est ensuite agité pendant 2 heures de 8°C à 21°C. La phase organique est séparée. La phase organique est lavée par l'eau (3 fois par 50 ml). Après concentration sous pression réduite (température du bain = 22-23°C, 220 mbar), de l'éther de pétrole (10 ml) est ajouté, le solvant est évaporé jusqu'à 8-10 ml, et la solution est maintenue à -18°C pendant 10-15 heures de façon à obtenir un précipité. Le précipité est filtré et lavé sur le filtre par le mélange de CH₂Cl₂ / éther de pétrole (2 ml / 6 ml) puis par l'éther de pétrole (2 fois 10 ml) et enfin séché pendant 10-15 heures sous pression atmosphérique à température ambiante. Un solide blanc (5,31 g, rendement 61 %) de point de fusion de 139°C est obtenu.

La pureté molaire est supérieure à 95 % mol (RMN ¹H).

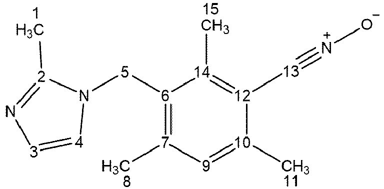

**[Table 12]**

| N° | δ¹H (ppm) | δ¹³C (ppm) |
|---|---|---|
| 1 | 2,3 | 12,6 |
| 2 | (-) | 143,6 |
| 3 | 6,59 | 126,1 |
| 4 | 6,72 | 117,1 |
| 5 | 4,99 | 43 |
| 6 | (-) | 130,6 |
| 7 | (-) | 140,7 |
| 8 | 2,16 | 19,2 |
| 9 | 7,12 | 129,9 |
| 10 | (-) | 141 |
| 11 | 2,34 | 20 |
| 12 | (-) | 112,1 |
| 13 | (-) | NI |
| 14 | (-) | 140,8 |
| 15 | 2,8 | 17,7 |

2.3 - Comparaison du procédé de synthèse de l'oxyde de nitrile (composé Xa) obtenu par transformation du composé (la) obtenu selon le procédé de l'invention (paragraphe 2.1) par rapport au procédé de synthèse de l'oxyde de nitrile (composé (Xa) selon le procédé de synthèse de l'art antérieur (paragraphe 2.2):

**[Table 13]**

| | | Procédé de synthèse du compos (Xa) de l'art antérieur (procédé 2.2) | Procédé de synthèse du composé (Xa) selon l'invention (procédé 2.1) |
|---|---|---|---|
| Nombre d'intermédiaire isolé | | 4 | 1 |
| rendement global | | 23 % | 52% |
| nombre de solvants utilisés | | 4 | 2 |
| Litres de solvant utilisés/kg de produit final | dichlorométhane | 117,9 | 8,9 |
| | Alcool (éthanol, isopropanol) | 12 | 6,2 |
| | éther de pétrole | 7,3 | 0 |
| | DMF | 2,2 | 0 |
| Litres dérivés aqueux (eau, HCl) utilisés/kg de produit final | | 311 | 63,5 |

De manière totalement inattendue, le procédé de synthèse du composé de formule (Xa) à partir du composé de formule (la) obtenu selon le procédé de l'invention permet une réduction des étapes de synthèse et du nombre d'intermédiaires isolés tout en améliorant le rendement. Il permet également une diminution du nombre de solvants utilisés, donc une simplification de manipulation par un opérateur. Enfin il permet une diminution des effluents et donc également de leurs traitements.

## Revendications

1. Procédé de synthèse d'un composé de formule (I) 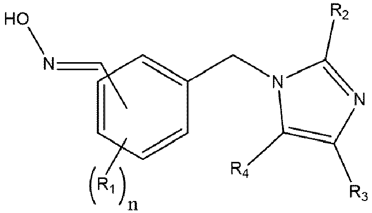 dans laquelle :
- R₁ représente, indépendamment les uns des autres, un alkyle en C1-C6, de préférence un alkyle en C1-C3 ;
- R₂ représente un atome d'hydrogène, un alkyle en C1-C12, de préférence un alkyle en C1-C6, plus préférentiellement un alkyle en C1-C3 ;
- R₃ et R₄, indépendamment l'un de l'autre, représente un atome d'hydrogène, un alkyle en C1-C12, (de préférence un alkyle en C1-C6, plus préférentiellement un alkyle en C1-C3) ou forment ensemble avec les atomes de carbone auxquels ils se rattachent un cycle, de préférence un cycle aromatique, de préférence le phényle; et
- n un nombre entier égal à 0, 1, 2, 3 ou 4 ; et ledit procédé de synthèse comprenant les étapes successives A1, A2, A3 suivantes :
• une étape d'obtention A1 d'un composé intermédiaire de formule (V) par une réaction de halogénométhylation d'un aldéhyde aromatique de formule (VI) en présence d'un agent de halogénométhylation selon le schéma réactionnel suivant : 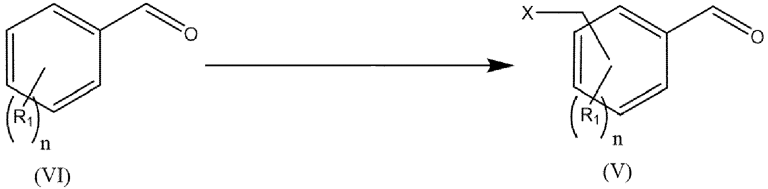 avec R₁ et n tels que définis ci-dessus ; et X étant un atome d'halogène, de préférence un atome de brome ou de chlore, plus préférentiellement un atome de chlore ;
• une étape d'obtention A2 d'un composé intermédiaire de formule (III) par substitution nucléophile du composé intermédiaire de formule (V) précédent avec un composé imidazole de formule (IV) selon le schéma réactionnel suivant : 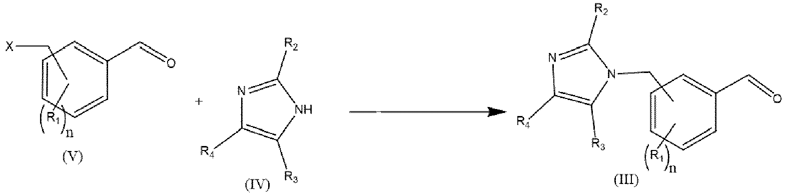 avec n, X, R₁, R₂, R₃ et R₄ tels que définis ci-dessus ;
• une étape d'obtention A3 du composé de formule (I) par une réaction de condensation du composé intermédiaire de formule (III) précédent avec l'hydroxylamine (II) selon le schéma réactionnel suivant : 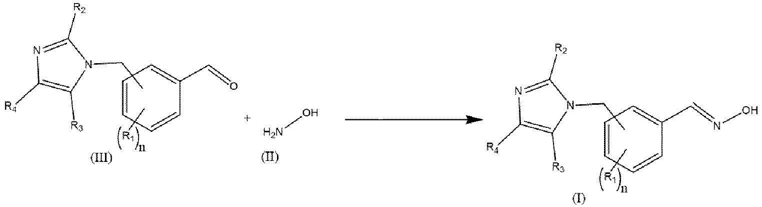
avec R₁, R₂, R₃, R₄ et n tels que définis ci-dessus ; et
les étapes A1, A2, A3 étant effectuées sans isolement d'au moins un composé intermédiaire choisi dans le groupe formé par le composé intermédiaire de formule (III) et le composé intermédiaire de formule (V).

2. Procédé selon la revendication 1, dans lequel l'agent d'halogénométhylation est préchauffé à une température T1 comprise dans un domaine allant de 23°C à 50°C, plus préférentiellement dans un domaine allant de 30°C à 45°C, avant sa mise en contact avec le composé de formule (VI).

3. Procédé selon la revendication 2, dans lequel le composé de formule (VI) est ajouté en une seule fois après le préchauffage de l'agent d'halogénométhylation.

4. Procédé selon l'une quelconque des revendications précédentes, dans lequel l'agent d'halogénométhylation est un mélange d'un donneur de formol et d'acide chlorhydrique.

5. Procédé selon la revendication 4, dans lequel la quantité de donneur de formol est comprise dans un domaine allant de 1 équivalent molaire à 7 équivalents molaires, de préférence de 1,2 équivalents molaires à 6 équivalents molaires, plus préférentiellement encore de 2 équivalents molaires à 6 équivalents molaires par rapport à la quantité de composé de formule (VI).

6. Procédé selon l'une quelconque des revendications précédentes, dans lequel l'étape A1 s'effectue à une température T2 comprise dans un domaine allant de 60°C à 100°C, plus préférentiellement dans un domaine allant de 70°C à 90°C.

7. Procédé selon l'une quelconque des revendications précédentes, dans lequel on ajoute un solvant organique S1 à la fin de l'étape A1 et on récupère la phase organique ; le solvant organique S1 étant choisi parmi les solvants halogénés.

8. Procédé selon l'une quelconque des revendications précédentes, dans lequel l'étape A2 s'effectue en présence d'un solvant organique S2 choisi dans le groupe constitué par les solvants halogénés, le diméthylsulfoxyde, l'acétone, les alcools tels que l'isopropanol, les éthers tels que le tétrahydrofurane, l'acétate d'éthyle ; préférentiellement le solvant organique S2 est un solvant organique halogéné, plus préférentiellement un solvant organique chloré.

9. Procédé selon la revendication 7 ou 8, dans lequel le solvant organique S2 est identique au solvant organique S1.

10. Procédé selon l'une quelconque des revendications précédentes, dans lequel la quantité de composé de formule (IV) est comprise dans un domaine allant de 1,1 équivalents molaires à 6 équivalents molaires, plus préférentiellement allant de 2 équivalents molaires à 4 équivalents molaires par rapport au composé de formule (V).

11. Procédé selon l'une quelconque selon l'une quelconque des revendications précédentes, dans lequel l'étape A2 s'effectue à une température qui est inférieure ou égale à la température de reflux du solvant organique S2.

12. Procédé selon l'une quelconque des revendications 8 à 11, dans lequel la quantité de solvant organique S2 est d'au moins 0,5 volume par rapport à 1 volume de solvant organique S1, plus préférentiellement est comprise dans un domaine allant de 1 volume à 10 volumes par rapport à 1 volume de solvant organique S1.

13. Procédé selon l'une quelconque des revendications précédentes, dans lequel l'étape A3 s'effectue dans un solvant organique S3 choisi dans le groupe constitué par les solvants halogénés, l'acétone, les alcools tels que l'isopropanol, les éthers tels que le tétrahydrofurane, l'acétate d'éthyle ; préférentiellement le solvant organique S3 un solvant organique halogéné, de préférence le solvant S3 est un solvant organique chloré.

14. Procédé selon la revendication 13, dans lequel le solvant organique S3 est identique au solvant organique S2.

15. Procédé de synthèse d'un composé de formule (X) ledit procédé comprenant les étapes suivantes :
• une étape (i) de synthèse du composé de formule (I) par le procédé de synthèse selon l'une quelconque des revendications précédentes,
• une étape (ii) de transformation du composé de formule (I) en composé de formule (X) en présence d'au moins un solvant organique S4 et d'au moins un oxydant selon le schéma réactionnel suivant : 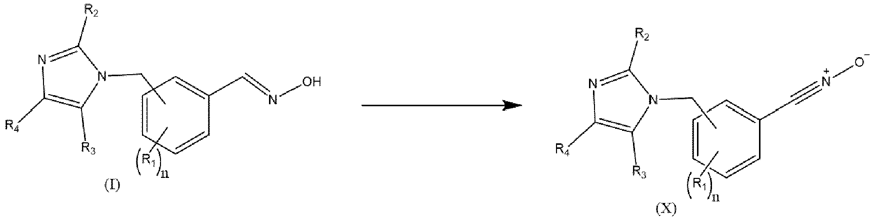 avec
- R₁ représente, indépendamment les uns des autres, un alkyle en C1-C6, de préférence un alkyle en C1-C3 ;
- R₂ représente un atome d'hydrogène, un alkyle en C1-C12, de préférence un alkyle en C1-C6, plus préférentiellement un alkyle en C1-C3 ;
- R₃ et R₄, indépendamment l'un de l'autre, représente un atome d'hydrogène, un alkyle en C1-C12, de préférence un alkyle en C1-C6, plus préférentiellement un alkyle en C1-C3, ou forment ensemble avec les atomes de carbone auxquels ils se rattachent un cycle, de préférence un cycle aromatique, plus préférentiellement le phényle et
- n un nombre entier égal à 0, 1, 2, 3 ou 4 ; et
• une étape (iii) de récupération du composé de formule (X).

## Patentansprüche

1. Verfahren zur Synthese einer Verbindung der Formel (I) 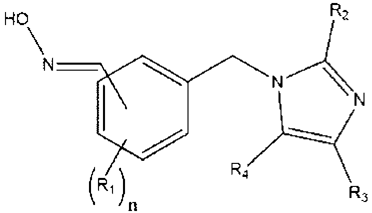 in der:
- R₁ unabhängig voneinander für ein C1-C6-Alkyl, vorzugsweise für ein C1-C3-Alkyl, steht;
- R₂ für ein Wasserstoffatom, ein C1-C12-Alkyl, vorzugsweise ein C1-C6-Alkyl, weiter bevorzugt ein C1-C3-Alkyl, steht;
- R₃ und R₄ unabhängig voneinander für ein Wasserstoffatom, ein C1-C12-Alkyl (vorzugsweise ein C1-C6-Alkyl, weiter bevorzugt ein C1-C3-Alkyl) stehen oder zusammen mit den Kohlenstoffatomen, an die sie gebunden sind, einen Ring, vorzugsweise einen aromatischen Ring, vorzugsweise Phenyl, bilden; und
- n eine ganze Zahl gleich 0, 1, 2, 3 oder 4 ist; und das Syntheseverfahren die folgenden aufeinanderfolgenden Schritte A1, A2, A3 umfasst:
• einen Schritt A1 des Erhalts einer Zwischenverbindung der Formel (V) durch eine Halogenmethylierungsreaktion eines aromatischen Aldehyds der Formel (VI) in Gegenwart eines Halogenmethylierungsmittels gemäß folgendem Reaktionsschema: 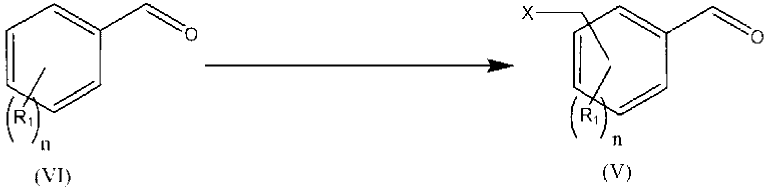 wobei R₁ und n wie oben definiert sind und wobei X ein Halogenatom, vorzugsweise ein Brom- oder Chloratom, weiter bevorzugt ein Chloratom, ist;
• einen Schritt A2 des Erhalts einer Zwischenverbindung der Formel (III) durch nucleophile Substitution der Zwischenverbindung der Formel (V) mit einer Imidazolverbindung der Formel (IV) gemäß folgendem Reaktionsschema: 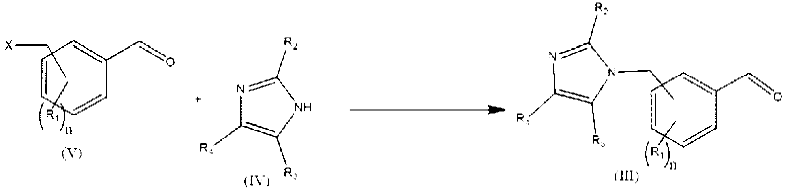 wobei n, X, R₁, R₂, R₃ und R₄ wie oben definiert sind;
• einen Schritt A3 des Erhalts der Verbindung der Formel (I) durch eine Kondensationsreaktion der vorhergehenden Zwischenverbindung der Formel (III) mit Hydroxylamin (II) gemäß folgendem Reaktionsschema: 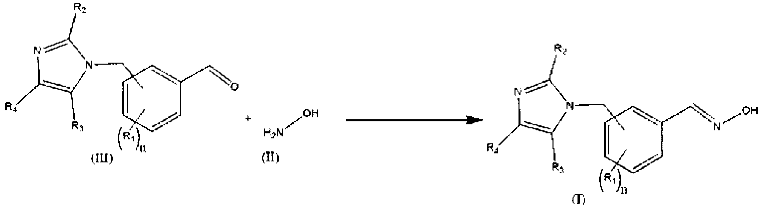
wobei R₁, R₂, R₃, R₄ und n wie oben definiert sind; und
wobei die Schritte A1, A2, A3 ohne Isolierung mindestens einer Zwischenverbindung, die aus der Gruppe bestehend aus der Zwischenverbindung der Formel (III) und der Zwischenverbindung der Formel (V) ausgewählt ist, durchgeführt werden.

2. Verfahren nach Anspruch 1, wobei das Halogenmethylierungsmittel auf eine Temperatur T1 in einem Bereich von 23 °C bis 50 °C, weiter bevorzugt in einem Bereich von 30 °C bis 45 °C, vorgewärmt wird, bevor es mit der Verbindung der Formel (VI) in Kontakt gebracht wird.

3. Verfahren nach Anspruch 2, wobei die Verbindung der Formel (VI) nach dem Vorwärmen des Halogenmethylierungsmittels auf einmal zugegeben wird.

4. Verfahren nach einem der vorhergehenden Ansprüche, wobei es sich bei dem Halogenmethylierungsmittel um eine Mischung von Formaldehyd-Donor und Salzsäure handelt.

5. Verfahren nach Anspruch 4, wobei die Menge an Formaldehyd-Donor in einem Bereich von 1 Moläquivalent bis 7 Moläquivalenten, vorzugsweise von 1,2 Moläquivalenten bis 6 Moläquivalenten, noch weiter bevorzugt von 2 Moläquivalenten bis 6 Moläquivalenten, bezogen auf die Menge der Verbindung der Formel (VI), liegt.

6. Verfahren nach einem der vorhergehenden Ansprüche, wobei der Schritt A1 bei einer Temperatur T2 in einem Bereich von 60 °C bis 100 °C, vorzugsweise in einem Bereich von 70 °C bis 90 °C, durchgeführt wird.

7. Verfahren nach einem der vorhergehenden Ansprüche, wobei am Ende von Schritt A1 ein organisches Lösungsmittel S1 zugegeben und die organische Phase zurückgewonnen wird, wobei das organische Lösungsmittel S1 aus halogenierten Lösungsmitteln ausgewählt wird.

8. Verfahren nach einem der vorhergehenden Ansprüche, wobei Schritt A2 in Gegenwart eines organischen Lösungsmittels S2, das aus der Gruppe bestehend aus halogenierten Lösungsmitteln, Dimethylsulfoxid, Aceton, Alkoholen wie Isopropanol, Ethern wie Tetrahydrofuran oder Essigsäureethylester ausgewählt wird, durchgeführt wird und es sich bei dem organischen Lösungsmittel S2 vorzugsweise um ein halogeniertes organisches Lösungsmittel, vorzugsweise ein chloriertes organisches Lösungsmittel, handelt.

9. Verfahren nach Anspruch 7 oder 8, wobei das organische Lösungsmittel S2 mit dem organischen Lösungsmittel S1 identisch ist.

10. Verfahren nach einem der vorhergehenden Ansprüche, wobei die Menge der Verbindung der Formel (IV) in einem Bereich von 1,1 Moläquivalenten bis 6 Moläquivalenten, weiter bevorzugt in einem Bereich von 2 Moläquivalenten bis 4 Moläquivalenten, bezogen auf die Verbindung der Formel (V), liegt.

11. Verfahren nach einem der vorhergehenden Ansprüche, wobei Schritt A2 bei einer Temperatur durchgeführt wird, die kleiner als die oder gleich der Rückflusstemperatur des organischen Lösungsmittels S2 ist.

12. Verfahren nach einem der Ansprüche 8 bis 11, wobei die Menge des organischen Lösungsmittels S2 mindestens 0,5 Volumen, bezogen auf 1 Volumen des organischen Lösungsmittels S1, beträgt und weiter bevorzugt in einem Bereich von 1 Volumen und 10 Volumina, bezogen auf 1 Volumen des organischen Lösungsmittels S1, beträgt.

13. Verfahren nach einem der vorhergehenden Ansprüche, wobei Schritt A3 in einem organischen Lösungsmittel S3, das aus der Gruppe bestehend aus halogenierten Lösungsmitteln, Aceton, Alkoholen wie Isopropanol, Ethern wie Tetrahydrofuran oder Essigsäureethylester, ausgewählt wird, durchgeführt wird, es sich bei dem organischen Lösungsmittel S3 vorzugsweise um ein halogeniertes organisches Lösungsmittel handelt und es sich bei dem Lösungsmittel S3 vorzugsweise um ein chloriertes organisches Lösungsmittel handelt.

14. Verfahren nach Anspruch 13, wobei das organische Lösungsmittel S3 mit dem organischen Lösungsmittel S2 identisch ist.

15. Verfahren zur Synthese einer Verbindung der Formel (X), wobei das Verfahren folgende Schritte umfasst:
• einen Schritt (i) der Synthese der Verbindung der Formel (I) durch das Syntheseverfahren nach einem der vorhergehenden Ansprüche,
• einen Schritt (ii) der Umwandlung der Verbindung der Formel (I) in eine Verbindung der Formel (X) in Gegenwart von mindestens einem organischen Lösungsmittel S4 und mindestens einem Oxidationsmittel gemäß folgendem Reaktionsschema: 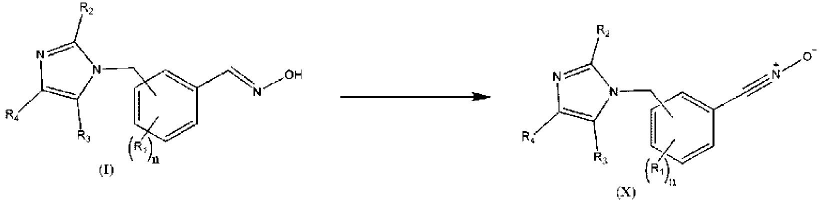 wobei
- R₁ unabhängig voneinander für ein C1-C6-Alkyl, vorzugsweise für ein C1-C3-Alkyl, steht;
- R₂ für ein Wasserstoffatom, ein C1-C12-Alkyl, vorzugsweise ein C1-C6-Alkyl, weiter bevorzugt ein C1-C3-Alkyl, steht;
- R₃ und R₄ unabhängig voneinander für ein Wasserstoffatom, ein C1-C12-Alkyl, vorzugsweise ein C1-C6-Alkyl, weiter bevorzugt ein C1-C3-Alkyl, stehen oder zusammen mit den Kohlenstoffatomen, an die sie gebunden sind, einen Ring, vorzugsweise einen aromatischen Ring, weiter bevorzugt Phenyl, bilden; und
- n eine ganze Zahl gleich 0, 1, 2, 3 oder 4 ist;
• einen Schritt (iii) der Gewinnung der Verbindung der Formel (X).

## Claims

1. Process for synthesizing a compound of formula (I) 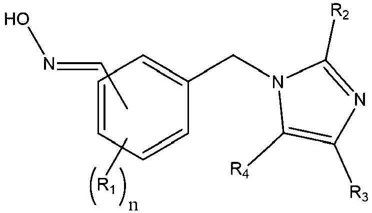 in which:
- R₁ represents, independently of one another, a C1-C6 alkyl, preferably a C1-C3 alkyl;
- R₂ represents a hydrogen atom, a C1-C12 alkyl, preferably a C1-C6 alkyl, more preferentially a C1-C3 alkyl;
- R₃ and R₄, independently of one another, represent a hydrogen atom, a C1-C12 alkyl, (preferably a C1-C6 alkyl, more preferentially a C1-C3 alkyl) or form, together with the carbon atoms to which they are attached, a ring, preferably an aromatic ring, preferably phenyl; and
- n is an integer equal to 0, 1, 2, 3 or 4; and said synthesis process comprising the following successive steps A1, A2 and A3:
• a step A1 of obtaining an intermediate compound of formula (V) via a halomethylation reaction of an aromatic aldehyde of formula (VI) in the presence of a halomethylation agent according to the following reaction scheme: 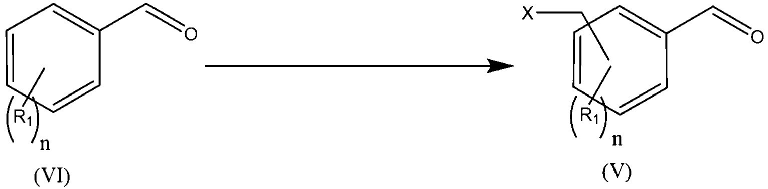 with R₁ and n as defined above; and X being a halogen atom, preferably a bromine or chlorine atom, more preferentially a chlorine atom;
• a step A2 of obtaining an intermediate compound of formula (III) via nucleophilic substitution of the preceding intermediate compound of formula (V) with an imidazole compound of formula (IV) according to the following reaction scheme: 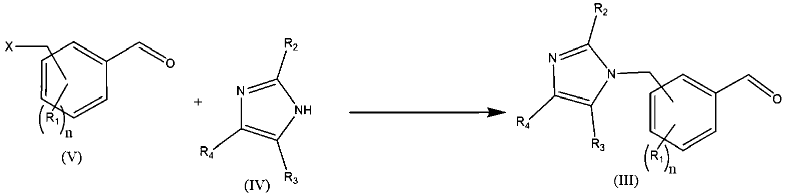 with n, X, R₁, R₂, R₃ and R₄ as defined above;
• a step A3 of obtaining the compound of formula (I) via a condensation reaction of the preceding intermediate compound of formula (III) with hydroxylamine (II) according to the following reaction scheme: 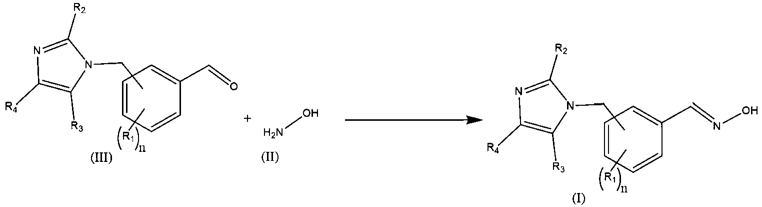
with R₁, R₂, R₃, R₄ and n as defined above; and
steps A1, A2, A3 being carried out without isolation of at least one intermediate compound chosen from the group formed by the intermediate compound of formula (III) and the intermediate compound of formula (V).

2. Process according to Claim 1, in which the halomethylation agent is preheated to a temperature T1 within a range extending from 23°C to 50°C, more preferentially within a range extending from 30°C to 45°C, before bringing it into contact with the compound of formula (VI).

3. Process according to Claim 2, in which the compound of formula (VI) is added all at once after the preheating of the halomethylation agent.

4. Process according to any one of the preceding claims, in which the halomethylation agent is a mixture of a formaldehyde donor and hydrochloric acid.

5. Process according to Claim 4, in which the amount of formaldehyde donor is within a range extending from 1 molar equivalent to 7 molar equivalents, preferably from 1.2 molar equivalents to 6 molar equivalents, more preferentially still from 2 molar equivalents to 6 molar equivalents, relative to the amount of compound of formula (VI).

6. Process according to any one of the preceding claims, in which step A1 is carried out at a temperature T2 within a range extending from 60°C to 100°C, more preferentially within a range extending from 70°C to 90°C.

7. Process according to any one of the preceding claims, in which an organic solvent S1 is added at the end of step A1 and the organic phase is recovered; the organic solvent S1 being chosen from halogenated solvents.

8. Process according to any one of the preceding claims, in which step A2 is carried out in the presence of an organic solvent S2 chosen from the group consisting of halogenated solvents, dimethyl sulfoxide, acetone, alcohols such as isopropanol, ethers such as tetrahydrofuran, ethyl acetate; preferentially the organic solvent S2 is a halogenated organic solvent, more preferentially a chlorinated organic solvent.

9. Process according to Claim 7 or 8, in which the organic solvent S2 is identical to the organic solvent S1.

10. Process according to any one of the preceding claims, in which the amount of compound of formula (IV) is within a range extending from 1.1 molar equivalents to 6 molar equivalents, more preferentially extending from 2 molar equivalents to 4 molar equivalents, relative to the compound of formula (V).

11. Process according to any one according to any one of the preceding claims, in which step A2 is carried out at a temperature which is less than or equal to the reflux temperature of the organic solvent S2.

12. Process according to any one of Claims 8 to 11, in which the amount of organic solvent S2 is at least 0.5 volume relative to 1 volume of organic solvent S1, more preferentially is within a range extending from 1 volume to 10 volumes relative to 1 volume of organic solvent S1.

13. Process according to any one of the preceding claims, in which step A3 is carried out in an organic solvent S3 chosen from the group consisting of halogenated solvents, acetone, alcohols such as isopropanol, ethers such as tetrahydrofuran, ethyl acetate; preferentially the organic solvent S3 a halogenated organic solvent, preferably the solvent S3 is a chlorinated organic solvent.

14. Process according to Claim 13, in which the organic solvent S3 is identical to the organic solvent S2.

15. Process for synthesizing a compound of formula (X), said process comprising the following steps:
• a step (i) of synthesizing the compound of formula (I) via the synthesis process according to any one of the preceding claims,
• a step (ii) of converting the compound of formula (I) into a compound of formula (X) in the presence of at least one organic solvent S4 and at least one oxidant according to the following reaction scheme: 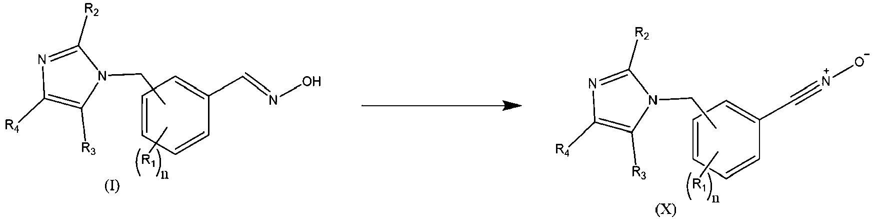 where
- R₁ represents, independently of one another, a C1-C6 alkyl, preferably a C1-C3 alkyl;
- R₂ represents a hydrogen atom, a C1-C12 alkyl, preferably a C1-C6 alkyl, more preferentially a C1-C3 alkyl;
- R₃ and R₄, independently of one another, represent a hydrogen atom, a C1-C12 alkyl, preferably a C1-C6 alkyl, more preferentially a C1-C3 alkyl, or form, together with the carbon atoms to which they are attached, a ring, preferably an aromatic ring, more preferentially phenyl, and
- n is an integer equal to 0, 1, 2, 3 or 4; and
• a step (iii) of recovering the compound of formula (X).
